# EUROPEAN PATENT APPLICATION

(11) **EP 4 458 356 A1**
(43) Date of publication of application: **06.11.2024**
(21) Application number: 22914797.0
(22) Date of filing: 27.12.2022
(51) Int. Cl.: A61K 31/41, A61K 31/27, A61P 27/02

(54) **COMPOSITION AND USE THEREOF IN PREPARATION OF MEDICAMENT FOR TREATING PRESBYOPIA**

(30) Priority: 28.12.2021 CN 202111622501
(71) Applicant: Shenyang Xingqi Pharmaceutical Co., Ltd., Shenyang, Liaoning 110167 (CN)
(72) Inventor: LIU, Jidong, Shenyang, Liaoning 110163 (CN); YANG, Qiang, Shenyang, Liaoning 110163 (CN); LI, Dan, Shenyang, Liaoning 110163 (CN); JIN, Yang, Shenyang, Liaoning 110163 (CN); ZHAO, Lili, Shenyang, Liaoning 110163 (CN)
(74) Representative: Patent 42
(86) International application number: PCT/CN2022/142375
(87) International publication number: WO 2023/125544

(57) **Abstract**

A composition and a use of the composition in the preparation of a medicament for treating eye diseases. The composition comprises 0.5-1.5% by weight of a muscarinic acetylcholine receptor M3 agonist; 0.005-0.5% by weight of naphazoline, a pharmaceutically acceptable salt thereof or a combination thereof; and 0.1-1% by weight of ketorolac tromethamine. While enhancing the effect of a muscarinic acetylcholine receptor M3 agonist such as a pilocarpine active ingredient in treating presbyopia, the composition effectively significantly reduces or even eliminates side effects caused by the pilocarpine active ingredient.

## Description

### Field of the Invention

The present invention relates to the field of medicine. Specifically, the present invention relates to a composition, particularly an ophthalmic preparation, preferably an eye drop or an eye spray, and the use of this composition in the preparation of a medicament for the treatment of ophthalmic disease such as presbyopia.

### Background of the Invention

The human eye has normal refractive power and central axial length, allowing distant objects to be seen immediately through the eye's optical system, wherein light passes through the eyes passively through muscle contraction. The focusing mechanism of the eyes in humans and primates is produced by changes in the shape and position of the lens, which are caused by the contraction of the ciliary muscle of the eye. From childhood, the lens gradually loses its ability to change shape and position by contraction of the ciliary muscles. From the perspective of optometry, hyperopia need to increase refractive power to see more clearly. However, at about 40 years old, presbyopia occurs with age, meaning that the normal eye cannot focus comfortably on objects close to the eye (within 40 centimeters). In China, this is commonly referred to as presbyopia.

In the eye, the ciliary muscle is under the control of the parasympathetic nervous system. The sympathetic nervous system plays a secondary (regulatory) role via its α and β receptors. One of the treatment mechanisms for presbyopia is to regulate the dilation of the iris and the function of the sphincter, which can change the pupil diameter. The iris sphincter is mainly under parasympathetic control via muscarinic receptors. M3 cholinoceptor agonists can increase contraction of the ciliary muscle and thus increase the refractive power of the eye. Therefore, it can be used to treat conditions such as presbyopia, mild hyperopia and irregular astigmatism, hyperopic accommodative esotropia, and glaucoma. Such receptor agonists mainly include acetylcholine, bethanechol chloride, carbachol, pilocarpidine or pilocarpine.

At present, the main treatment for presbyopia is M3 cholinoceptor agonists, wherein pilocarpine is used as the main active ingredients. Pilocarpine has been used as a separate medication for the treatment of presbyopia and mild hyperopia, but has not achieved very satisfactory results because the local application concentration less than 0.5% has minimal effect on accommodation and thus is ineffective, while the concentration higher than 0.5% is intolerable due to side effects such as redness of the eyes, eye pain, migraine, and headache. In addition, when the concentration of pilocarpine is sufficient to effectively improve hyperopia, this myopic appearance of the eye results in a significant decrease in the far vision of the eye (Gilmartin et al., 1995, Ophthalmic and Physiological Optics, Pergamon Press, Oxford, GB, 15(5):475-479). In current treatment protocols, there is no medication that can achieve satisfactory therapeutic effects while addressing the side effects as redness of the eyes, eye pain, migraine, and headache caused by the use of pilocarpine. The present invention provides a solution to effectively alleviate the side effects of pilocarpine on the basis of achieving satisfactory therapeutic effects.

The application CN112272558A (application number 201980037652.3) from the American company Allergan disclosed a eye drop composition containing only pilocarpine hydrochloride as the active ingredient for the treatment of presbyopia. The pilocarpine hydrochloride eye drop composition comprises about 1.25% w/v of pilocarpine hydrochloride, about 1.0% w/v of boric acid, about 0.015% w/v of sodium citrate dihydrate, about 0.08% w/v of sodium chloride, and about 0.0075% w/v of benzalkonium chloride. The results of clinical trials conducted with this eye drop composition showed that doses of pilocarpine at concentrations ≥1% and <1.5% were most effective in improving reading ability in presbyopic patients, without significantly increasing the incidence of adverse reactions.

The pilocarpine hydrochloride eye drop composition contains only pilocarpine hydrochloride as an active ingredient, and the incidence of side effects is only not significantly increased. There are no other active ingredients to suppress the side effects associated with the use of pilocarpine hydrochloride. The occurrence of redness of the eyes, eye pain, migraine, and headache is due to the use of pilocarpine, and other specific active ingredients need to be used to suppress these adverse reactions one by one, so as to significantly reduce the incidence of these adverse reactions.

The application CN104093404A (application number 201280056271.8) of the American Allergan Company disclosed a compound preparation composition containing M3 cholinoceptor agonist, oxymetazoline, and NSAID (nonsteroidal anti-inflammatory drugs) for the treatment of presbyopia. The composition comprises 0.5%-1.5% w/w of muscarinic acetylcholine receptor M3 agonist, 0.02%-0.1% w/w of oxymetazoline, and COX-2 selective NSAID (including one or more of meloxicam, celecoxib, rofecoxib, valdecoxib, parecoxib, etoricoxib, nimesulide, etodolac, and nabumetone). The preparation composition it used for the treatment of presbyopia, mild hyperopia, irregular astigmatism, hyperopic accommodative esotropia, or the enhancement or intensification of delaying, reversing or altering the aging process of the lens and its surrounding tissues in subjects.

The compound preparation composition effectively alleviates the side effects caused by the use of pilocarpine by specifically incorporating oxymetazoline and a nonsteroidal anti-inflammatory drug (NSAID). Oxymetazoline is an α receptor agonist with an imidazoline group, which has a vasoconstrictive effect and is used to relieve redness of the eyes. The COX-2 selective NSAID (this patent includes one or more of meloxicam, celecoxib, rofecoxib, valdecoxib, parecoxib, etoricoxib, nimesulide, etodolac, and nabumetone) can selectively inhibit COX-2, that means, can inhibit cyclooxygenase-2, which produces prostaglandins, thereby inhibiting the development of inflammation. Although the NSAID included in the patent can alleviate inflammation, it does not have a significant effect on reducing headaches caused by the use of pilocarpine. Therefore, although it can alleviate certain side effects, it has not yet reached an ideal state.

Therefore, for the pilocarpine-type eye drops composition, it is still necessary to be able to maintain or even enhance the therapeutic effect of the pilocarpine-type active ingredient on presbyopia while effectively significantly reducing or even eliminating the side effects caused by the pilocarpine-type active ingredient.

### Summary of the Invention

One objective of the present invention is to provide a composition that synergistically enhances the efficacy of pilocarpine-type active ingredients in the treatment of presbyopia.

Another objective of the present invention is to provide a composition that can enhance the efficacy of pilocarpine while significantly reducing the side effects associated with pilocarpine.

Another object of the present invention is to provide the use of the composition in the preparation of a medicament for the treatment of ophthalmic disease such as presbyopia.

In response to the above technical problems, the inventor conducted in-depth research and made the following discoveries, thereby completing the present invention.

In particular, the present invention can be implemented as follows.
1. A composition comprising, relative to the total weight of the composition:
   0.5-1.5 weight% of muscarinic acetylcholine receptor M3 agonist;
   0.005-0.5 weight% of naphazoline, its pharmaceutically acceptable salt, or a combination thereof; and
   0.1-1 weight% of ketorolac tromethamine.
2. The composition of item 1, wherein the composition is an ophthalmic preparation, preferably an eye drop or an eye spray.
3. The composition of any one of items 1-2, wherein the muscarinic acetylcholine receptor M3 agonist comprises at least one selected from the group consisting of acetylcholine, bethanechol chloride, carbachol, oxotremorine, pilocarpidine, pilocarpine, a pharmaceutically acceptable salt thereof, or a combination thereof;
   Preferably, the muscarinic acetylcholine receptor M3 agonist comprises at least one selected from pilocarpine, its pharmaceutically acceptable salt, or a combination thereof;
   Preferably, the muscarinic acetylcholine receptor M3 agonist is pilocarpine, pilocarpine hydrochloride, pilocarpine nitrate, or a combination thereof, preferably pilocarpine nitrate.
4. The composition of any one of items 1-3, wherein the pharmaceutically acceptable salt of naphazoline is naphazoline hydrochloride.
5. The composition of any one of items 1-4, wherein the composition comprises water, preferably injection water, as a liquid carrier.
6. The composition of any one of items 1-5, wherein the composition does not comprise a bacteriostatic agent.
7. The composition of any one of items 1-6, wherein the composition does not comprise an α receptor agonist with an imidazoline group other than naphazoline, its pharmaceutically acceptable salt, or a combination thereof.
8. The composition of claim 7, wherein the composition does not comprise an α receptor agonist other than naphazoline, its pharmaceutically acceptable salt, or a combination thereof.
9. The composition of any one of items 1-8, wherein the composition does not comprise any other active pharmaceutical ingredient (API) other than a muscarinic acetylcholine receptor M3 agonist, naphazoline, its pharmaceutically acceptable salt, or a combination thereof, and ketorolac tromethamine.
10. The composition of any one of items 1-9, wherein the composition does not comprise sodium chloride.
11. The composition of any one of items 1-10, wherein the composition has a pH of 3.0-8.5 at room temperature, preferably the composition has a pH of 4.0-6.0 at room temperature, and preferably the composition has a pH of 4.5-5.5 at room temperature.
12. The composition of item 1, wherein the composition further comprises an osmotic pressure regulator, preferably in an amount of 0.1-2.0% relative to the total weight of the composition.
13. The composition of item 12, wherein the osmotic pressure regulator comprises one or more selected from the group consisting of propylene glycol, glycerol, sodium chloride, glycerin, glucose, boric acid, and borax.
14. The composition of item 13, wherein the osmotic pressure regulator is boric acid, preferably 0.5-1.5 weight% of boric acid relative to the total weight of the composition.
15. The composition of any one of items 1-14, wherein the composition further comprises a surfactant;
   Preferably, the surfactant is 0.1-1 weight% relative to the total weight of the composition;
   Preferably, the surfactant comprises one or more selected from the group consisting of polyoxyethylene (20) hydrogenated castor oil, polyoxyethylene (40) hydrogenated castor oil, polyoxyethylene (60) hydrogenated castor oil, polysorbate 80, Span, poloxamer, propylene glycol, tyloxapol, and nonoxynol;
   Preferably, the surfactant is polyoxyethylene (40) hydrogenated castor oil.
16. The composition of any one of items 1-15, wherein the composition further comprises a stabilizer;
   Preferably, the stabilizer comprises one or more selected from the group consisting of disodium edetate, EDTA, and sodium bisulfite,
   Preferably, the stabilizer is 0.005-0.05 weight% of sodium citrate relative to the total weight of the composition, and/or 0.005-0.05 weight% of disodium edetate relative to the total weight of the composition.
17. The composition of any one of items 1-16, wherein the composition further comprises a pH adjusting agent;
   Preferably, the pH adjusting agent comprises one or more selected from the group consisting of citric acid, phosphoric acid, acetic acid, hydrochloric acid and other acids, sodium hydroxide, potassium hydroxide and other alkalis.
18. The composition of any one of items 1-17, wherein the pH adjusting agent comprises:
   0-0.1 weight% of citric acid and/or 0-0.1 weight% of sodium hydroxide relative to the total weight of the composition.
19. The composition of any one of items 1-18, wherein the composition comprises:
   0.5-1.5 weight% of pilocarpine nitrate;
   0.005-0.5 weight% of naphazoline and/or naphazoline hydrochloride;
   0.1-1 weight% of ketorolac tromethamine;
   0.5-1.5 weight% of boric acid;
   0.005-0.05 weight% of sodium citrate;
   0.005-0.05 weight% of disodium edetate;
   0.1-1 weight% of polyoxyethylene (40) hydrogenated castor oil;
   0-0.1 weight% of citric acid;
   0-0.1 weight% of sodium hydroxide; and
   remaining injection water.
20. Use of the composition of any one of items 1-19 in the preparation of a medicament for the treatment of ophthalmic disease, such as presbyopia.

The inventors were surprised to find that when a muscarinic acetylcholine receptor M3 agonist such as pilocarpine-type active ingredient is used in combination with naphazoline or its pharmaceutically acceptable salt (such as naphazoline hydrochloride) and ketorolac tromethamine in specific amounts, there is a synergistic effect between naphazoline or its pharmaceutically acceptable salt (such as naphazoline hydrochloride) and ketorolac tromethamine, which synergistically improves the efficacy of the composition. This not only effectively treats presbyopia but also significantly reduces or even eliminates the side effects such as redness of the eyes, eye pain, migraine, and headache caused by the muscarinic acetylcholine receptor M3 agonist such as pilocarpine-type active ingredient. For example, by using naphazoline or its pharmaceutically acceptable salt (such as naphazoline hydrochloride) and ketorolac tromethamine in combination, the composition of the present invention can achieve better therapeutic effects for presbyopia with a lower amount of the muscarinic acetylcholine receptor M3 agonist such as pilocarpine-type active ingredient and/or lower side effects (such as redness of the eyes, eye pain, migraine, and headache) with a higher amount of the muscarinic acetylcholine receptor M3 agonist such as pilocarpine-type active ingredient and/or longer effective treatment duration (continuous effective treatment for a longer period of time). In addition, the composition of the present invention has good druggability.

The independent claims and dependent claims describe the specific and preferred features of the invention. Features from dependent claims can be combined with features from the independent claims or other dependent claims as appropriate.

The present invention will now be further described. In the following paragraphs, different aspects of the invention are defined in more detail. Unless specifically indicated to the contrary, each aspect so defined may be combined with any other aspect or aspects. In particular, any feature or statement that is identified as preferred or advantageous may be combined with any other feature or statement that is identified as preferred or advantageous.

### Detailed Description of the Invention

When describing the present invention, unless the context otherwise dictates, the terms used are to be interpreted as follows.

In this article, the term "pharmaceutically acceptable salt" refers to inorganic acid salt or organic acid salt, wherein the inorganic acids can be hydrochloric acid, hydrobromic acid, nitric acid, phosphoric acid, sulfuric acid, and perchloric acid; organic acids can be acetic acid, oxalic acid, maleic acid, tartaric acid, citric acid, succinic acid, or malonic acid. Other pharmaceutically acceptable salt include adipic acid salt, alginic acid salt, ascorbic acid salt, aspartic acid salt, benzenesulfonic acid salt, benzoic acid salt, bisulfate, boric acid salt, butyric acid salt, camphor salt, camphor sulfonic acid salt, cyclopentanepropionate, digluconate, dodecyl sulfate, esylate, formate, fumarate, glucoheptonate, glycerophosphate, glucoheptonate, hemisulfate, heptanoic acid salt, hexanoic acid salt, iodide, 2-hydroxyethylsulfonate, lactobionate, lactate, lauric acid salt, lauryl sulfate, malic acid salt, maleic acid salt, malonic acid salt, methanesulfonate, 2-naphthalenesulfonate, nicotinic acid salt, nitrate, oleic acid salt, oxalic acid salt, palmitic acid salt, pamoate, pectic acid salt, persulfate, 3-phenylpropionic acid salt, phosphate, picric acid salt, trimethylacetic acid salt, propionic acid salt, stearic acid salt, succinate, sulfate, tartaric acid salt, thiocyanate, p-toluenesulfonate, undecylenic acid salt and valeric acid salt.

In this article, the term "Naphazoline hydrochloride" is sometimes referred to as "Naphazoline hydrochloride salt", and it can be interchangeably used; in this article, the term "Pilocarpine nitrate" is sometimes referred to as 'Pilocarpine nitrate salt", and it can be interchangeably used.

Unless otherwise defined, all terms used in the disclosure of the present invention, including technical and scientific terms, have meanings that are commonly understood by those of ordinary skill in the art to which the present invention belongs.

In the following paragraphs, different aspects of the present invention are defined in more detail. Unless specifically indicated to the contrary, each aspect so defined may be combined with any other aspect or aspects. In particular, any feature identified as preferred or advantageous may be combined with any other feature or features identified as preferred or advantageous.

Throughout this specification, references to "an embodiment" or "an implementation" mean that the specific features, structures, or characteristics described in connection with the embodiment are included in at least one embodiment of the present invention. Therefore, references to "in one embodiment" or "in an implementation" in different places throughout this specification do not necessarily all refer to the same embodiment, but may all refer to the same embodiment. Moreover, in one or more embodiments, specific features, structures, or characteristics may be combined in any suitable manner as will be apparent to those skilled in the art from this disclosure. Furthermore, although some embodiments described herein comprise some features that are also comprised in other embodiments, they do not comprise all of the features comprised in those other embodiments. However, the combination of features of different embodiments is intended to be within the scope of the present invention and forms different embodiments as will be understood by those skilled in the art. For example, in the appended claims and the following description, any of the embodiments may be used in any combination.

The term "comprise" as used herein is synonymous with "contains" or "includes" and is inclusive or open-ended and does not exclude additional, unlisted members, elements, or method steps. It will be appreciated that the term "comprise" as used herein includes the term "consisting of".

The numerical ranges recited by endpoints include all integers included within the range and, as appropriate, fractions (e.g., 1-5 may include 1, 2, 3, 4 when referring to, for example, the number of elements, and may also include 1.5, 2, 2.75, and 3.80 when referring to, for example, measurements). The recitation of endpoints also includes the endpoint values themselves (e.g., 1.0-5.0 includes both 1.0 and 5.0). Any numerical ranges recited herein are intended to encompass all sub-ranges included within them.

In this document, when numerical values are mentioned, unless otherwise specified, the numerical values mentioned should be understood to disclose the numerical values themselves, and also encompass changes in the numerical values of plus or minus 10% or less, preferably plus or minus 5% or less, more preferably plus or minus 1% or less, as long as such changes are suitable for use in the disclosed invention.

The invention will now be described in detail. Unless specifically indicated to the contrary, each statement and embodiment of the invention thus defined may be combined with any other statement and/or embodiment. In particular, any feature specified as preferred or advantageous may be combined with any other feature or features or statement specified as preferred or advantageous. In this regard, the invention is particularly obtained by any combination of any one of the following numbered statements and embodiments or any one or more thereof with any other aspect and/or embodiment.

A first aspect of the present invention relates to a composition comprising:
Muscarinic acetylcholine receptor M3 agonist;
Naphazoline, its pharmaceutically acceptable salt, or a combination thereof, and
Ketorolac tromethamine.

In one embodiment, the composition is an ophthalmic preparation, preferably an eye drop or an eye spray.

In one embodiment, the muscarinic acetylcholine receptor M3 agonist is present in an amount of 0.5-1.5 weight% relative to the total weight of the composition, for example 0.50%, 0.51%, 0.52%, 0.53%, 0.54%, 0.55%, 0.56%, 0.57%, 0.58%, 0.59%, 0.60%, 0.61%, 0.62%, 0.63%, 0.64%, 0.65%, 0.66%, 0.67%, 0.68%, 0.69%, 0.70%, 0.71%, 0.72%, 0.73%, 0.74%, 0.75%, 0.76%, 0.77%, 0.78%, 0.79%, 0.80%, 0.81%, 0.82%, 0.83%, 0.84%, 0.85%, 0.86%, 0.87%, 0.88%, 0.89%, 0.90%, 0.91%, 0.92%, 0.93%, 0.94%, 0.95%, 0.96%, 0.97%, 0.98%, 0.99%, 1.00%, 1.01%, 1.02%, 1.03%, 1.04%, 1.05%, 1.06%, 1.07%, 1.08%, 1.09%, 1.10%, 1.11%, 1.12%, 1.13%, 1.14%, 1.15%, 1.16%, 1.17%, 1.18%, 1.19%, 1.20%, 1.21%, 1.22%, 1.23%, 1.24%, 1.25%, 1.26%, 1.27%, 1.28%, 1.29%, 1.30%, 1.31%, 1.32%, 1.33%, 1.34%, 1.35%, 1.36%, 1.37%, 1.38%, 1.39%, 1.40%, 1.41%, 1.42%, 1.43%, 1.44%, 1.45%, 1.46%, 1.47%, 1.48%, 1.49%, 1.50%, or a range defined by any two of them.

In one embodiment, the muscarinic acetylcholine receptor M3 agonist including at least one selected from the group consisting of acetylcholine, bethanechol chloride, carbachol, oxotremorine, pilocarpidine, pilocarpine, a pharmaceutically acceptable salt thereof, or a combination thereof.

In one embodiment, the muscarinic acetylcholine receptor M3 agonist is pilocarpine, its pharmaceutically acceptable salt, or a combination thereof.

In one embodiment, the muscarinic acetylcholine receptor M3 agonist is pilocarpine, pilocarpine hydrochloride, pilocarpine nitrate, or a combination thereof, preferably pilocarpine nitrate.

In one embodiment, naphazoline, its pharmaceutically acceptable salt, or a combination thereof is present in an amount of 0.005-0.5 weight% relative to the total weight of the composition, for example 0.005%, 0.006%, 0.007%, 0.008%, 0.009%, 0.010%, 0.011%, 0.012%, 0.013%, 0.014%, 0.015%, 0.016%, 0.017%, 0.018%, 0.019%, 0.020%, 0.021%, 0.022%, 0.023%, 0.024%, 0.025%, 0.026%, 0.027%, 0.028%, 0.029%, 0.030%, 0.031%, 0.032%, 0.033%, 0.034%, 0.035%, 0.036%, 0.037%, 0.038%, 0.039%, 0.040%, 0.041%, 0.042%, 0.043%, 0.044%, 0.045%, 0.046%, 0.047%, 0.048%, 0.049%, 0.050%, 0.051%, 0.052%, 0.053%, 0.054%, 0.055%, 0.056%, 0.057%, 0.058%, 0.059%, 0.060%, 0.061%, 0.062%, 0.063%, 0.064%, 0.065%, 0.066%, 0.067%, 0.068%, 0.069%, 0.070%, 0.071%, 0.072%, 0.073%, 0.074%, 0.075%, 0.076%, 0.077%, 0.078%, 0.079%, 0.080%, 0.081%, 0.082%, 0.083%, 0.084%, 0.085%, 0.086%, 0.087%, 0.088%, 0.089%, 0.090%, 0.091%, 0.092%, 0.093%, 0.094%, 0.095%, 0.096%, 0.097%, 0.098%, 0.099%, 0.100%, 0.105%, 0.110%, 0.115%, 0.120%, 0.125%, 0.130%, 0.135%, 0.140%, 0.145%, 0.150%, 0.155%, 0.160%, 0.165%, 0.170%, 0.175%, 0.180%, 0.185%, 0.190%, 0.195%, 0.200%, 0.205%, 0.210%, 0.215%, 0.220%, 0.225%, 0.230%, 0.235%, 0.240%, 0.245%, 0.250%, 0.255%, 0.260%, 0.265%, 0.270%, 0.275%, 0.280%, 0.285%, 0.290%, 0.295%, 0.300%, 0.305%, 0.310%, 0.315%, 0.320%, 0.325%, 0.330%, 0.335%, 0.340%, 0.345%, 0.350%, 0.355%, 0.360%, 0.365%, 0.370%, 0.375%, 0.380%, 0.385%, 0.390%, 0.395%, 0.400%, 0.405%, 0.410%, 0.415%, 0.420%, 0.425%, 0.430%, 0.435%, 0.440%, 0.445%, 0.450%, 0.455%, 0.460%, 0.465%, 0.470%, 0.475%, 0.480%, 0.485%, 0.490%, 0.495%, 0.500%, or a range defined by any two of them.

In one embodiment, the pharmaceutically acceptable salt of naphazoline is naphazoline hydrochloride.

In one embodiment, ketorolac tromethamine is present in an amount of 0.1-1 weight% relative to the total weight of the composition, for example 0.10%, 0.11%, 0.12%, 0.13%, 0.14%, 0.15%, 0.16%, 0.17%, 0.18%, 0.19%, 0.20%, 0.21%, 0.22%, 0.23%, 0.24%, 0.25%, 0.26%, 0.27%, 0.28%, 0.29%, 0.30%, 0.31%, 0.32%, 0.33%, 0.34%, 0.35%, 0.36%, 0.37%, 0.38%, 0.39%, 0.40%, 0.41%, 0.42%, 0.43%, 0.44%, 0.45%, 0.46%, 0.47%, 0.48%, 0.49%, 0.50%, 0.51%, 0.52%, 0.53%, 0.54%, 0.55%, 0.56%, 0.57%, 0.58%, 0.59%, 0.60%, 0.61%, 0.62%, 0.63%, 0.64%, 0.65%, 0.66%, 0.67%, 0.68%, 0.69%, 0.70%, 0.71%, 0.72%, 0.73%, 0.74%, 0.75%, 0.76%, 0.77%, 0.78%, 0.79%, 0.80%, 0.81%, 0.82%, 0.83%, 0.84%, 0.85%, 0.86%, 0.87%, 0.88%, 0.89%, 0.90%, 0.91%, 0.92%, 0.93%, 0.94%, 0.95%, 0.96%, 0.97%, 0.98%, 0.99%, 1.00%, or a range defined by any two of them.

In one embodiment, the composition does not comprise a bacteriostatic agent.

In one embodiment, the composition does not comprise an α receptor agonist with an imidazoline group other than naphazoline, its pharmaceutically acceptable salt, or a combination thereof.

In one embodiment, the composition does not comprise an α receptor agonist other than naphazoline, its pharmaceutically acceptable salt, or a combination thereof.

In one embodiment, the composition does not comprise any other active pharmaceutical ingredient (API) other than muscarinic acetylcholine receptor M3 agonist, naphazoline, its pharmaceutically acceptable salt, or a combination thereof, and ketorolac tromethamine.

In one embodiment, the composition comprises water, preferably injection water, as a liquid carrier.

In one embodiment, the composition may further comprise a surfactant.

In one embodiment, the surfactant comprises one or more selected from the group consisting of polyoxyethylene (20) hydrogenated castor oil, polyoxyethylene (40) hydrogenated castor oil, polyoxyethylene (60) hydrogenated castor oil, polysorbate 80, Span, poloxamer, propylene glycol, tyloxapol, and nonoxynol.

In one embodiment, the surfactant (for example, polyoxyethylene (40) hydrogenated castor oil) is present in an amount of 0.1-1% relative to the total weight of the composition, for example 0.10%, 0.11%, 0.12%, 0.13%, 0.14%, 0.15%, 0.16%, 0.17%, 0.18%, 0.19%, 0.20%, 0.21%, 0.22%, 0.23%, 0.24%, 0.25%, 0.26%, 0.27%, 0.28%, 0.29%, 0.30%, 0.31%, 0.32%, 0.33%, 0.34%, 0.35%, 0.36%, 0.37%, 0.38%, 0.39%, 0.40%, 0.41%, 0.42%, 0.43%, 0.44%, 0.45%, 0.46%, 0.47%, 0.48%, 0.49%, 0.50%, 0.51%, 0.52%, 0.53%, 0.54%, 0.55%, 0.56%, 0.57%, 0.58%, 0.59%, 0.60%, 0.61%, 0.62%, 0.63%, 0.64%, 0.65%, 0.66%, 0.67%, 0.68%, 0.69%, 0.70%, 0.71%, 0.72%, 0.73%, 0.74%, 0.75%, 0.76%, 0.77%, 0.78%, 0.79%, 0.80%, 0.81%, 0.82%, 0.83%, 0.84%, 0.85%, 0.86%, 0.87%, 0.88%, 0.89%, 0.90%, 0.91%, 0.92%, 0.93%, 0.94%, 0.95%, 0.96%, 0.97%, 0.98%, 0.99%, 1.00%, or a range defined by any two of them.

In one embodiment, the composition may further comprise an osmotic pressure regulator.

In one embodiment, the osmotic pressure regulator comprises one or more selected from the group consisting of propylene glycol, glycerol, sodium chloride, glycerin, glucose, boric acid, and borax.

In one embodiment, the osmotic pressure regulator is present in an amount of 0.1-2.0% relative to the total weight of the composition, for example 0.10%, 0.15%, 0.20%, 0.25%, 0.30%, 0.35%, 0.40%, 0.45%, 0.50%, 0.55%, 0.60%, 0.65%, 0.70%, 0.75%, 0.80%, 0.85%, 0.90%, 0.95%, 1.00%, 1.05%, 1.10%, 1.15%, 1.20%, 1.25%, 1.30%, 1.35%, 1.40%, 1.45%, 1.50%, 1.55%, 1.60%, 1.65%, 1.70%, 1.75%, 1.80%, 1.85%, 1.90%, 1.95%, 2.00%, or a range defined by any two of them.

In one embodiment, the osmotic pressure regulator comprises boric acid or is boric acid, which is present in an amount of 0.5-1.5% relative to the total weight of the composition, for example 0.50%, 0.51%, 0.52%, 0.53%, 0.54%, 0.55%, 0.56%, 0.57%, 0.58%, 0.59%, 0.60%, 0.61%, 0.62%, 0.63%, 0.64%, 0.65%, 0.66%, 0.67%, 0.68%, 0.69%, 0.70%, 0.71%, 0.72%, 0.73%, 0.74%, 0.75%, 0.76%, 0.77%, 0.78%, 0.79%, 0.80%, 0.81%, 0.82%, 0.83%, 0.84%, 0.85%, 0.86%, 0.87%, 0.88%, 0.89%, 0.90%, 0.91%, 0.92%, 0.93%, 0.94%, 0.95%, 0.96%, 0.97%, 0.98%, 0.99%, 1.00%, 1.01%, 1.02%, 1.03%, 1.04%, 1.05%, 1.06%, 1.07%, 1.08%, 1.09%, 1.10%, 1.11%, 1.12%, 1.13%, 1.14%, 1.15%, 1.16%, 1.17%, 1.18%, 1.19%, 1.20%, 1.21%, 1.22%, 1.23%, 1.24%, 1.25%, 1.26%, 1.27%, 1.28%, 1.29%, 1.30%, 1.31%, 1.32%, 1.33%, 1.34%, 1.35%, 1.36%, 1.37%, 1.38%, 1.39%, 1.40%, 1.41%, 1.42%, 1.43%, 1.44%, 1.45%, 1.46%, 1.47%, 1.48%, 1.49%, 1.50%, or a range defined by any two of them.

In one embodiment, the osmotic pressure regulator may further comprise propylene glycol, wherein the propylene glycol is present in an amount of 0.1-1% relative to the total weight of the composition, for example 0.10%, 0.11%, 0.12%, 0.13%, 0.14%, 0.15%, 0.16%, 0.17%, 0.18%, 0.19%, 0.20%, 0.21%, 0.22%, 0.23%, 0.24%, 0.25%, 0.26%, 0.27%, 0.28%, 0.29%, 0.30%, 0.31%, 0.32%, 0.33%, 0.34%, 0.35%, 0.36%, 0.37%, 0.38%, 0.39%, 0.40%, 0.41%, 0.42%, 0.43%, 0.44%, 0.45%, 0.46%, 0.47%, 0.48%, 0.49%, 0.50%, 0.51%, 0.52%, 0.53%, 0.54%, 0.55%, 0.56%, 0.57%, 0.58%, 0.59%, 0.60%, 0.61%, 0.62%, 0.63%, 0.64%, 0.65%, 0.66%, 0.67%, 0.68%, 0.69%, 0.70%, 0.71%, 0.72%, 0.73%, 0.74%, 0.75%, 0.76%, 0.77%, 0.78%, 0.79%, 0.80%, 0.81%, 0.82%, 0.83%, 0.84%, 0.85%, 0.86%, 0.87%, 0.88%, 0.89%, 0.90%, 0.91%, 0.92%, 0.93%, 0.94%, 0.95%, 0.96%, 0.97%, 0.98%, 0.99%, 1.00%, or a range defined by any two of them.

In one embodiment, the composition may further comprise a stabilizer.

In one embodiment, the stabilizer comprises one or more selected from the group consisting of disodium edetate, EDTA, and sodium bisulfite.

In one embodiment, the stabilizer (such as disodium edetate) is present in an amount of 0.005-0.05 weight% relative to the total weight of the composition, for example 0.005%, 0.006%, 0.007%, 0.008%, 0.009%, 0.010%, 0.011%, 0.012%, 0.013%, 0.014%, 0.015%, 0.016%, 0.017%, 0.018%, 0.019%, 0.020%, 0.021%, 0.022%, 0.023%, 0.024%, 0.025%, 0.026%, 0.027%, 0.028%, 0.029%, 0.030%, 0.031%, 0.032%, 0.033%, 0.034%, 0.035%, 0.036%, 0.037%, 0.038%, 0.039%, 0.040%, 0.041%, 0.042%, 0.043%, 0.044%, 0.045%, 0.046%, 0.047%, 0.048%, 0.049%, 0.050%, or a range defined by any two of them.

In one embodiment, the composition may further comprise a pH buffering agent.

In one embodiment, the pH buffering agent comprises one or more selected from the group consisting of phosphate and citrate.

In one embodiment, the pH buffering agent (e.g., sodium citrate) is present in an amount of 0.005-0.05% relative to the total weight of the composition, for example 0.005%, 0.006%, 0.007%, 0.008%, 0.009%, 0.010%, 0.011%, 0.012%, 0.013%, 0.014%, 0.015%, 0.016%, 0.017%, 0.018%, 0.019%, 0.020%, 0.021%, 0.022%, 0.023%, 0.024%, 0.025%, 0.026%, 0.027%, 0.028%, 0.029%, 0.030%, 0.031%, 0.032%, 0.033%, 0.034%, 0.035%, 0.036%, 0.037%, 0.038%, 0.039%, 0.040%, 0.041%, 0.042%, 0.043%, 0.044%, 0.045%, 0.046%, 0.047%, 0.048%, 0.049%, 0.050%, or a range defined by any two of them.

In one embodiment, the composition may further comprise a pH adjusting agent.

In one embodiment, the pH adjusting agent comprises one or more selected from the group consisting of citric acid, phosphoric acid, acetic acid, hydrochloric acid and other acids, and sodium hydroxide, potassium hydroxide and other alkalis.

In one embodiment, the pH adjusting agent is present in an amount of 0-0.5% relative to the total weight of the composition, for example 0%, 0.01%, 0.02%, 0.03%, 0.04%, 0.05%, 0.06%, 0.07%, 0.08%, 0.09%, 0.1%, 0.11%, 0.12%, 0.13%, 0.14%, 0.15%, 0.16%, 0.17%, 0.18%, 0.19%, 0.2%, 0.21%, 0.22%, 0.23%, 0.24%, 0.25%, 0.26%, 0.27%, 0.28%, 0.29%, 0.3%, 0.31%, 0.32%, 0.33%, 0.34%, 0.35%, 0.36%, 0.37%, 0.38%, 0.39%, 0.4%, 0.41%, 0.42%, 0.43%, 0.44%, 0.45%, 0.46%, 0.47%, 0.48%, 0.49%, 0.5%, or a range defined by any two of them.

In one embodiment, the pH adjusting agent comprises citric acid, and the citric acid is present in an amount of 0-0.1% relative to the total weight of the composition, for example 0%, 0.01%, 0.02%, 0.03%, 0.04%, 0.05%, 0.06%, 0.07%, 0.08%, 0.09%, 0.1%, or a range defined by any two of them.

In one embodiment, the pH adjusting agent comprises sodium hydroxide, and the amount of sodium hydroxide is 0-0.1% relative to the total weight of the composition, for example 0%, 0.01%, 0.02%, 0.03%, 0.04%, 0.05%, 0.06%, 0.07%, 0.08%, 0.09%, 0.1%, or a range defined by any two of them.

In one embodiment, the pH adjusting agent (e.g., citric acid and/or sodium hydroxide) is present in an amount such that the composition has a pH of 3.0-8.5 at room temperature, for example 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, or a range defined by any two of them.

In one embodiment, the pharmaceutical composition may have a pH at room temperature as follows: 3.0-8.5, for example 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, or a range defined by any two of them.

In one embodiment, the pharmaceutical composition has a pH of 3.0-7.0, preferably 3.5-6.5, preferably 4.0-6.0, preferably 4.5-5.5, for example 5.0 at room temperature.

In one embodiment, the composition comprises:
0.5-1.5 weight% of pilocarpine nitrate;
0.005-0.5 weight% of naphazoline and/or naphazoline hydrochloride;
0.1-1 weight% of ketorolac tromethamine;
0.1-1 weight% of polyoxyethylene (40) hydrogenated castor oil;
0.5-1.5 weight% of boric acid;
0.005-0.05 weight% of sodium citrate;
0.005-0.05 weight% of disodium edetate;
0-0.1 weight% of citric acid;
0-0.1 weight% of sodium hydroxide; and
remaining injection water.

A second aspect of the invention relates to the use of the composition as described above for the preparation of a medicament for the treatment of ophthalmic disease.

In one embodiment, the ophthalmic disease include presbyopia, mild hyperopia, irregular astigmatism, hyperopic accommodative esotropia, and glaucoma; preferably, the ophthalmic disease is presbyopia.

In this article, "ophthalmic disease" refers to conditions, diseases, or disorders that affect or involve the eye or a part or area of the eye, including the eyeball and the tissues and fluids that make up the eyeball, the muscles around the eye (such as the oblique and rectus muscles), and parts of the optic nerve within or adjacent to the eyeball. Examples of ophthalmic disease include presbyopia and mild hyperopia, irregular astigmatism, hyperopic accommodative esotropia, and glaucoma.

The inventors of the present invention were surprised to find that when a muscarinic acetylcholine receptor M3 agonist such as pilocarpine-type active ingredients is used in combination with naphazoline or its pharmaceutically acceptable salt (such as naphazoline hydrochloride) and ketorolac tromethamine in specific amounts, there is a synergistic effect between naphazoline hydrochloride and ketorolac tromethamine, which synergistically improves the efficacy, significantly reduces the side effects, and has good druggability, being able to effectively treat presbyopia while more significantly reducing or even eliminating the side effects such as redness of the eyes, eye pain, migraine, and headache caused by muscarinic acetylcholine receptor M3 agonists such as pilocarpine-type active ingredients. For example, by using naphazoline or its pharmaceutically acceptable salt (such as naphazoline hydrochloride) and ketorolac tromethamine in combination, the composition of the present invention can achieve better treatment effect of presbyopia with lower amounts of muscarinic acetylcholine receptor M3 agonists such as pilocarpine-type active ingredients and/or lower side effects (such as redness of the eyes, eye pain, migraine, and headache) with higher amounts of muscarinic acetylcholine receptor M3 agonists and/or longer effective treatment duration (obtaining continuous effective treatment for a longer period of time) compared to the eye drop composition of the prior art.

The composition of the present invention can achieve sustained and effective treatment of presbyopia at the ideal time, effectively alleviating the redness of the eyes and headache caused by pilocarpine, greatly enhancing the compliance of elderly patients with medication. Moreover, while addressing the compatibility issues of existing drugs, the composition can further reduce the irritation of the composition on the eyes of presbyopic patients after removing the bacteriostatic agent.

The above-mentioned are merely exemplary embodiments of the present invention. It should be noted that those skilled in the art can make improvements without departing from the inventive concept of the present invention, but these improvements are all within the scope of protection of the present invention.

### Example

### Experiment 1

Prepare the solution as shown in Table 1-1-1 and conduct efficacy and irritation tests.

**Table 1-1-1: Components of Different Groups**

| Group | Pilocarpi ne nitrate (weight %) | Naphazo line hydrochl oride (weight %) | Ketorolac tromethami ne (weight%) | Boric acid (weight %) | Sodium citrate (weight %) | Disodiu m edetate (weight %) | Polyoxyeth ylene 40 hydrogenate d castor oil (weight%) |
|---|---|---|---|---|---|---|---|
| Example 1 | 1 | 0.05 | 0.5 | 1 | 0.015 | 0.015 | 0.5 |
| Comparati ve Example 1 | 1 | 0 | 0 | 1 | 0.015 | 0.015 | 0.5 |
| Comparati ve Example 2 | 1 | 0.05 | 0 | 1 | 0.015 | 0.015 | 0.5 |
| Comparati ve Example 3 | 1 | 0 | 0.5 | 1 | 0.015 | 0.015 | 0.5 |

Preparation: Prepare 1L of each of the samples of the Examples and Comparative Examples according to the weight percentages listed in Table 1-1-1. Add sodium citrate, disodium edetate, boric acid, and about 800ml of water, stirring to dissolve; then add polyoxyethylene 40 hydrogenated castor oil, stirring to dissolve; then add pilocarpine nitrate, naphazoline hydrochloride, and ketorolac tromethamine separately, stirring to dissolve, adjust the pH to 5.0, and add water to 1L.

### Experiment 1-1: Efficacy Test

The efficacy of the 4 formulations was evaluated using rabbit as the animal model, with the pupil diameter as the index. 20 rabbits were randomly divided into 4 groups, and 50µL of test drug solution was applied to the right eyes of the rabbits in each group according to the Example 1, Comparative Example 1, Comparative Example 2, and Comparative Example 3. The lateral pupil diameter (unit: mm) was measured with an ophthalmic optical coherence tomography for anterior segment of the eye (anterior segment OCT, CASIA2 model, Tomey company) at different time points after administration. The average diameter of each group was calculated as the result of the pupil diameter in the table below.

The results are as follows:

**Table 1-1-2: The pupil diameter after administration (mm)**

| Group | Before administr ation | 15min | 120min | 240min | 300min | 330min | 360min |
|---|---|---|---|---|---|---|---|
| Example 1 | 8.46 | 5.17 | 6.19 | 6.45 | 6.72 | 6.94 | 7.08 |
| Comparati ve Example 1 | 8.77 | 5.30 | 6.54 | 7.20 | 7.41 | 8.58 | 8.67 |
| Comparati ve Example 2 | 8.58 | 5.46 | 6.60 | 7.36 | 7.42 | 8.42 | 8.29 |
| Comparati ve Example 3 | 8.39 | 5.34 | 6.31 | 6.49 | 6.95 | 7.01 | 7.27 |

According to the test results in Table 1-1-2, the reduction in the pupil diameter after administration compared to before administration can be calculated for Example 1 and Comparative Examples 1-3.

**Table 1-1-3: The reduction in pupil diameter after Administration(mm)**

| Group | 15min | 120min | 240min | 300min | 330min | 360min |
|---|---|---|---|---|---|---|
| Example 1 | 3.29 | 2.27 | 2.01 | 1.74 | 1.52 | 1.38 |
| Comparati ve Example 1 | 3.47 | 2.23 | 1.57 | 1.36 | 0.19 | 0.10 |
| Comparati ve Example 2 | 3.12 | 1.98 | 1.22 | 1.16 | 0.16 | 0.29 |
| Comparati ve Example 3 | 3.05 | 2.08 | 1.90 | 1.44 | 1.38 | 1.12 |

Through further calculation, it can be known that: compared with Comparative Example 1 in which only the pilocarpine nitrate was used, the change of the pupil diameter reduction value after administration in the case of Comparative Example 2 using pilocarpine nitrate and naphazoline hydrochloride, Comparative Example 3 using pilocarpine nitrate and ketorolac tromethamine, and Example 1 using a combination of pilocarpine nitrate, naphazoline hydrochloride, and ketorolac tromethamine.

**Table 1-1-4: The change of the pupil diameter reduction value after administration compared to Comparative Example 1 (mm)**

| Group | 15min | 120min | 240min | 300min | 330min | 360min |
|---|---|---|---|---|---|---|
| Example 1 | -0.18 | +0.04 | +0.44 | +0.38 | +1.33 | +1.28 |
| Comparati ve Example 2 | -0.35 | -0.25 | -0.35 | -0.20 | -0.03 | +0.19 |
| Comparati ve Example 3 | -0.42 | -0.15 | +0.33 | +0.08 | +1.19 | +1.02 |
| Comparati ve Example 2+3 | -0.77 | -0.40 | -0.02 | -0.12 | +1.16 | +1.21 |

According to the above calculation results, it can be known that:

### (Comparative Example 1)

In Comparative Example 1, only pilocarpine nitrate was used. The pupil diameter of the rabbit's eye significantly decreased at 15min after administration compared to the preadministration value (the reduction value of the pupil diameter reached 3.47 at 15 min), and then gradually decreased (the reduction value of the pupil diameter decreased to 2.23 at 120 min; the reduction value of the pupil diameter decreased to 1.57 at 240 min; the reduction value of the pupil diameter decreased to 1.36 at 300 min). At 5.5 hours after administration, the pupil diameter of the rabbit's eye almost completely recovered (the reduction value of the pupil diameter decreased to 0.19 at 330 min), indicating that the effect of reducing the pupil diameter of the rabbit's eye almost completely resolved at 5.5 hours after administration;

### (Comparative Example 2)

In Comparative Example 2, pilocarpine nitrate and naphazoline hydrochloride were used, wherein
- The change in the reduction value of the rabbit's eye pupil diameter at 15 min after administration was -0.35 compared to Comparative Example 1 using only pilocarpine nitrate (i.e., the pharmacodynamic effect of pilocarpine nitrate in reducing the pupil diameter of the rabbit's eye was weakened),
- The change in the reduction value of the rabbit's eye pupil diameter at 2 hours after administration was -0.25 compared to Comparative Example 1 using only pilocarpine nitrate,
- The change in the reduction value of the rabbit's eye pupil diameter at 4 hours after administration was -0.35 compared to Comparative Example 1 using only pilocarpine nitrate,
- The change in the reduction value of the rabbit's eye pupil diameter at 5 hours after administration was -0.20 compared to Comparative Example 1 using only pilocarpine nitrate,
- The change in the reduction value of the rabbit's eye pupil diameter at 5.5 hours after administration was -0.03 compared to Comparative Example 1 using only pilocarpine nitrate,
- The change in the reduction value of the rabbit's eye pupil diameter at 6 hours after administration was -0.19 compared to Comparative Example 1 using only pilocarpine nitrate,

It can be seen that when using naphazoline hydrochloride alone, the efficacy of pilocarpine nitrate was weakened within 5.5 hours after administration;

### (Comparative Example 3)

In Comparative Example 3, pilocarpine nitrate and ketorolac tromethamine were used, wherein
- The change in the reduction value of the rabbit's eye pupil diameter at 15 min after administration was -0.42 compared to Comparative Example 1 using only pilocarpine nitrate(i.e., the pharmacodynamic effect of pilocarpine nitrate in reducing the pupil diameter of the rabbit's eye was weakened),
- The change in the reduction value of the rabbit's eye pupil diameter at 2 hours after administration was -0.15 compared to Comparative Example 1 using only pilocarpine nitrate,
- The change in the reduction value of the rabbit's eye pupil diameter at 4 hours after administration was +0.33 compared to Comparative Example 1 using only pilocarpine nitrate(i.e., the pharmacodynamic effect of pilocarpine nitrate in reducing the pupil diameter of the rabbit's eye was enhanced).
- The change in the reduction value of the rabbit's eye pupil diameter at 5 hours after administration was +0.08 compared to Comparative Example 1 using only pilocarpine nitrate,
- The change in the reduction value of the rabbit's eye pupil diameter at 5.5 hours after administration was +1.19 compared to Comparative Example 1 using only pilocarpine nitrate,
- The change in the reduction value of the rabbit's eye pupil diameter at 6 hours after administration was +1.02 compared to Comparative Example 1 using only pilocarpine nitrate,

It can be seen that when ketorolac tromethamine is used alone, the efficacy of pilocarpine nitrate is weakened within 2 hours after administration, and to some extent, it is enhanced at 4 to 6 hours after administration;

### (Example 1)

In Example 1, pilocarpine nitrate, naphazoline hydrochloride, and ketorolac tromethamine were used in combination, wherein
- The change in the reduction value of the rabbit's eye pupil diameter at 15 minutes after administration was -0.18 compared to Comparative Example 1 using only pilocarpine nitrate, and this effect was significantly better than the sum of the effect of using naphazoline hydrochloride alone and the effect of using ketorolac tromethamine alone (Comparative Example 2 + Comparative Example 3), which was -0.77.
- The change in the reduction value of the rabbit's eye pupil diameter at 2 hours after administration was +0.04 compared to Comparative Example 1 using only pilocarpine nitrate (i.e., the pharmacodynamic effect of pilocarpine nitrate in reducing the pupil diameter of the rabbit's eye was enhanced), and this effect was significantly better than the sum of the effect of using naphazoline hydrochloride alone and the effect of using ketorolac tromethamine alone (Comparative Example 2 + Comparative Example 3), which was -0.40,
- The change in the reduction value of the rabbit's eye pupil diameter at 4 hours after administration was +0.44 compared to Comparative Example 1 using only pilocarpine nitrate, and this effect was significantly better than the sum of the effect of using naphazoline hydrochloride alone and the effect of using ketorolac tromethamine alone (Comparative Example 2 + Comparative Example 3), which was -0.02,
- The change in the reduction value of the rabbit's eye pupil diameter at 5 hours after administration was +0.38 compared to Comparative Example 1 using only pilocarpine nitrate, and this effect was significantly better than the sum of the effect of using naphazoline hydrochloride alone and the effect of using ketorolac tromethamine alone (Comparative Example 2 + Comparative Example 3), which was -0.12,
- The change in the reduction value of the rabbit's eye pupil diameter at 5.5 hours after administration was +1.33 compared to Comparative Example 1 using only pilocarpine nitrate, and this effect was significantly better than the sum of the effect of using naphazoline hydrochloride alone and the effect of using ketorolac tromethamine alone (Comparative Example 2 + Comparative Example 3), which was +1.16,
- The change in the reduction value of the rabbit's eye pupil diameter at 6 hours after administration was +1.28 compared to Comparative Example 1 using only pilocarpine nitrate, and this effect was better than the sum of the effect of using naphazoline hydrochloride alone and the effect of using ketorolac tromethamine alone (Comparative Example 2 + Comparative Example 3), which was +1.21.

It can be seen that the combination of naphazoline hydrochloride and ketorolac tromethamine can significantly enhance the efficacy of pilocarpine nitrate, and the enhancement effect is significantly better than the sum of the effects of using naphazoline hydrochloride alone and the effect of using ketorolac tromethamine alone within 6 hours after administration.

The results above indicate that the combination of naphazoline hydrochloride and ketorolac tromethamine has an significant synergistic effect in enhancing efficacy.

### Experiment 1-2: Comparison Test of Conjunctival Congestion

The efficacy of the 4 formulations was evaluated using rabbits as subjects, with the degree of conjunctival congestion as the index. 20 rabbits were randomly divided into 4 groups, and one drop of test drug solution was applied to the right eye of each group's rabbits according to the Example 1, Comparative Example 1, Comparative Example 2, and Comparative Example 3. The degree of conjunctival congestion in the treated eyes was observed at different time points after administration. The evaluation criteria for congestion degree are shown in the following table:

**Table 1-2-1 Congestion Degree**

| Severity | describe |
|---|---|
| 0 | Normal: Conjunctival vessels are clear and easy to observe |
| 1 | Mild: Mild redness of the palpebral conjunctiva, with some radiation around the limbus of the cornea |
| 2 | Moderate: Bright deep red of the palpebral conjunctiva, covering at least 75% of the limbus of the cornea, with individual vessels not easily discernible |
| 3 | Severe: Dark red, with congestion of the bulbar conjunctiva and palpebral conjunctiva |

The results are as follows:

**Table 1-2-2: Example 1 Group**

| N | Before administr ation | 1min | 2min | 5min | 10min | 30min | 60min |
|---|---|---|---|---|---|---|---|
| 1 | 0 | 0 | 0 | 0 | 0 | 0 | 0 |
| 2 | 0 | 1 | 1 | 0 | 0 | 0 | 0 |
| 3 | 0 | 0 | 0 | 0 | 0 | 0 | 0 |
| 4 | 0 | 0 | 0 | 0 | 0 | 0 | 0 |
| 5 | 0 | 0 | 0 | 0 | 0 | 0 | 0 |
| average | 0 | 0.2 | 0.2 | 0 | 0 | 0 | 0 |

**Table 1-2-3: Comparative Example 1 Group**

| N | Before administr ation | 1min | 2min | 5min | 10min | 30min | 60min |
|---|---|---|---|---|---|---|---|
| 1 | 0 | 1 | 1 | 1 | 1 | 0 | 0 |
| 2 | 0 | 2 | 2 | 1 | 1 | 1 | 0 |
| 3 | 0 | 1 | 1 | 1 | 1 | 0 | 0 |
| 4 | 0 | 1 | 2 | 1 | 1 | 1 | 0 |
| 5 | 0 | 2 | 2 | 2 | 1 | 0 | 0 |
| average | 0 | 1.4 | 1.6 | 1.2 | 1 | 0.4 | 0 |

**Table 1-2-4: Comparative Example 2 Group**

| N | Before administr ation | 1min | 2min | 5min | 10min | 30min | 60min |
|---|---|---|---|---|---|---|---|
| 1 | 0 | 1 | 1 | 0 | 0 | 0 | 0 |
| 2 | 0 | 0 | 0 | 0 | 0 | 0 | 0 |
| 3 | 0 | 2 | 1 | 1 | 0 | 0 | 0 |
| 4 | 0 | 0 | 1 | 0 | 0 | 0 | 0 |
| 5 | 0 | 0 | 0 | 0 | 0 | 0 | 0 |
| average | 0 | 0.6 | 0.6 | 0.2 | 0 | 0 | 0 |

**Table 1-2-5: Comparative Example 3 Group**

| N | Before administr ation | 1min | 2min | 5min | 10min | 30min | 60min |
|---|---|---|---|---|---|---|---|
| 1 | 0 | 1 | 1 | 0 | 0 | 0 | 0 |
| 2 | 0 | 0 | 0 | 0 | 0 | 0 | 0 |
| 3 | 0 | 1 | 1 | 1 | 0 | 0 | 0 |
| 4 | 0 | 1 | 1 | 1 | 0 | 0 | 0 |
| 5 | 0 | 2 | 1 | 1 | 0 | 0 | 0 |
| average | 0 | 1 | 0.8 | 0.6 | 0 | 0 | 0 |

Based on the results of the above tests, it can be concluded that:
In Comparative Example 1, only pilocarpine nitrate was used, and 5 animals all showed mild to moderate conjunctival congestion within 1min after administration, which lasted for 30min;
In Comparative Example 2, pilocarpine nitrate and naphazoline hydrochloride were used, wherein
   - The average value of the degree of conjunctival congestion at 1 min after administration was reduced by 0.8 (0.6-1.4) compared to Comparative Example 1 using only pilocarpine nitrate,
   - The average value of the degree of conjunctival congestion at 2 min after administration was reduced by 1.0 (0.6-1.6) compared to Comparative Example 1 using only pilocarpine nitrate,
   - The average value of the degree of conjunctival congestion at 5 min after administration was reduced by 1.0 (0.2-1.2) compared to Comparative Example 1 using only pilocarpine nitrate, and
   - The conjunctival congestion completely resolved at 10min after administration.

It can be seen that the use of naphazoline hydrochloride alone can reduce the side effects of pilocarpine nitrate to a certain extent.

In Comparative Example 3, pilocarpine nitrate and ketorolac tromethamine were used, wherein
- The average value of the degree of conjunctival congestion at 1 min after administration was reduced by 0.4 (1.0-1.4) compared to Comparative Example 1 using only pilocarpine nitrate,
- The average value of the degree of conjunctival congestion at 2 min after administration was reduced by 0.8 (0.8-1.6) compared to Comparative Example 1 using only pilocarpine nitrate,
- The average value of the degree of conjunctival congestion at 5 min after administration was reduced by 0.6 (0.6-1.2) compared to Comparative Example 1 using only pilocarpine nitrate, and
- The conjunctival congestion completely resolved at 10 minutes after administration.

It can be seen that the use of ketorolac tromethamine alone can reduce the side effects of pilocarpine nitrate to a certain extent.

In Example 1, pilocarpine nitrate, naphazoline hydrochloride, and ketorolac tromethamine were used in combination, wherein
- The average value of the degree of conjunctival congestion at 1 min after administration was reduced by 1.2 (0.2-1.4) compared to Comparative Example 1 using only pilocarpine nitrate,
- The average value of the degree of conjunctival congestion at 2 min after administration was reduced by 1.4 (0.2-1.6) compared to Comparative Example 1 using only pilocarpine nitrate, and
- The conjunctival congestion completely resolved at 5 minutes after administration, and
- After administration, only mild conjunctival congestion (severity 1) was observed, and no moderate conjunctival congestion (severity 2) was observed, which was significantly better than either of Comparative Examples 2 and 3.
- The degree of conjunctival congestion at each time point was lower than either of the Comparative Examples 2 and 3.
- The number of animals with conjunctival congestion was lower than either of Comparative Examples 2 and 3.

It can be seen that the combination of naphazoline hydrochloride and ketorolac tromethamine can synergistically enhance the efficacy of pilocarpine nitrate while significantly reducing its side effects. The enhancement effect is superior to that of naphazoline hydrochloride alone and that of ketorolac tromethamine alone, and the effect of reducing the side effects is superior to that of naphazoline hydrochloride alone and that of ketorolac tromethamine alone.

The results above indicate that the combination of naphazoline hydrochloride and ketorolac tromethamine can enhance the efficacy of pilocarpine nitrate while significantly reducing its side effects.

### Experiment 1-3: Comparison Test of Pain:

To truly understand the pain caused by the 4 formulations mentioned above, we conducted an experimental study using human subjects. 20 individuals were randomly divided into 4 groups, and one drop of test drug solution of Example 1, Comparative Example 1, Comparative Example 2 and Comparative Example 3 was applied to the right eye of each subject in accordance with their group. The pain levels were observed at different time points after administration, and the pain level evaluation criteria are shown in the table below:

**Table 1-3-1 Pain Level**

| Severity | describe |
|---|---|
| 0 | Normal: No pain. |
| 1 | Mild: Slight pain, usually intermittent, does not affect the use of the eye. |
| 2 | Moderate: Persistent pain that has interfered with normal use of the eye and |
| | requires medical intervention. |
| 3 | Severe: Persistent severe pain, ineffective with medical intervention. |

The results are as follows:

**Table 1-3-2: Example 1 Group**

| N | Before administr ation | 1min | 2min | 5min | 10min | 30min | 60min |
|---|---|---|---|---|---|---|---|
| 1 | 0 | 0 | 0 | 0 | 0 | 0 | 0 |
| 2 | 0 | 0 | 0 | 0 | 0 | 0 | 0 |
| 3 | 0 | 1 | 0 | 0 | 0 | 0 | 0 |
| 4 | 0 | 0 | 1 | 0 | 0 | 0 | 0 |
| 5 | 0 | 0 | 0 | 0 | 0 | 0 | 0 |
| average | 0 | 0.2 | 0.2 | 0 | 0 | 0 | 0 |

**Table 1-3-3: Comparative Example 1 Group**

| N | Before administr ation | 1min | 2min | 5min | 10min | 30min | 60min |
|---|---|---|---|---|---|---|---|
| 1 | 0 | 1 | 1 | 1 | 1 | 0 | 0 |
| 2 | 0 | 2 | 2 | 1 | 1 | 1 | 0 |
| 3 | 0 | 2 | 1 | 1 | 0 | 0 | 0 |
| 4 | 0 | 1 | 2 | 1 | 1 | 1 | 0 |
| 5 | 0 | 2 | 2 | 2 | 1 | 1 | 1 |
| average | 0 | 1.6 | 1.6 | 1.2 | 0.8 | 0.6 | 0.2 |

**Table 1-3-4: Comparative Example 2 Group**

| N | Before administr ation | 1min | 2min | 5min | 10min | 30min | 60min |
|---|---|---|---|---|---|---|---|
| 1 | 0 | 0 | 1 | 1 | 0 | 0 | 0 |
| 2 | 0 | 1 | 1 | 1 | 0 | 0 | 0 |
| 3 | 0 | 2 | 1 | 1 | 1 | 0 | 0 |
| 4 | 0 | 1 | 1 | 0 | 0 | 0 | 0 |
| 5 | 0 | 2 | 1 | 1 | 1 | 1 | 0 |
| average | 0 | 1.2 | 1.0 | 0.8 | 0.4 | 0.2 | 0 |

**Table 1-3-5: Comparative Example 3 Group**

| N | Before administr ation | 1min | 2min | 5min | 10min | 30min | 60min |
|---|---|---|---|---|---|---|---|
| 1 | 0 | 0 | 0 | 0 | 0 | 0 | 0 |
| 2 | 0 | 0 | 0 | 0 | 0 | 0 | 0 |
| 3 | 0 | 0 | 0 | 0 | 0 | 0 | 0 |
| 4 | 0 | 1 | 1 | 1 | 0 | 0 | 0 |
| 5 | 0 | 1 | 1 | 1 | 1 | 0 | 0 |
| average | 0 | 0.4 | 0.4 | 0.4 | 0.2 | 0 | 0 |

Based on the results of the above tests, it can be concluded that:
In Comparative Example 1, only pilocarpine nitrate was used, and 5 people experienced mild to moderate pain for 60 min after administration;
In Comparative Example 2, pilocarpine nitrate and naphazoline hydrochloride were used, wherein
   - The average value of pain level at 1 min after administration was reduced by 0.4 (1.2-1.6) compared to Comparative Example 1 using only pilocarpine nitrate,
   - The average value of pain level at 2 min after administration was reduced by 0.6 (1.0-1.6) compared to Comparative Example 1 using only pilocarpine nitrate,
   - The average value of pain level at 5 min after administration was reduced by 0.4 (0.8-1.2) compared to Comparative Example 1 using only pilocarpine nitrate,
   - The average value of pain level at 10 min after administration was reduced by 0.4 (0.4-0.8) compared to Comparative Example 1 using only pilocarpine nitrate,
   - The average value of pain level at 30 min after administration was reduced by 0.4 (0.2-0.6) compared to Comparative Example 1 using only pilocarpine nitrate, and
   - The pain completely resolved at 30min after administration.

It can be seen that the use of naphazoline hydrochloride alone can reduce the side effects of pilocarpine nitrate to a certain extent.

In Comparative Example 3, pilocarpine nitrate and ketorolac tromethamine were used, wherein
- The average value of pain level at 1 min after administration was reduced by 1.2 (0.4-1.6) compared to Comparative Example 1 using only pilocarpine nitrate,
- The average value of pain level at 2 min after administration was reduced by 1.2 (0.4-1.6) compared to Comparative Example 1 using only pilocarpine nitrate,
- The average value of pain level at 5 min after administration was reduced by 0.8 (0.4-1.2) compared to Comparative Example 1 using only pilocarpine nitrate,
- The average value of pain level at 10 min after administration was reduced by 0.6 (0.2-0.8) compared to Comparative Example 1 using only pilocarpine nitrate, and
- The pain completely resolved at 10 minutes after administration.

It can be seen that the use of ketorolac tromethamine alone can reduce the side effects of pilocarpine nitrate to a certain extent.

In Example 1, pilocarpine nitrate, naphazoline hydrochloride, and ketorolac tromethamine were used in combination, wherein
- The average value of pain level at 1 min after administration was reduced by 1.4 (0.2-1.6) compared to Comparative Example 1 using only pilocarpine nitrate,
- The average value of pain level at 2 min after administration was reduced by 1.4 (0.2-1.6) compared to Comparative Example 1 using only pilocarpine nitrate, and
- The pain completely resolved at 5 minutes after administration, and
- After administration, only mild pain (severity of 1) was observed, and no moderate pain (severity of 2) was observed, which was significantly better than either of the Comparative Examples 2 and 3.
- The pain levels at each time point were lower than either of Comparative Examples 2 and 3.
- The number of people experiencing pain was lower than either of the Comparative Examples 2 and 3.

It can be seen that the combination of naphazoline hydrochloride and ketorolac tromethamine can synergistically enhance the efficacy of pilocarpine nitrate while significantly reducing its side effects. The enhancement effect is superior to that of naphazoline hydrochloride alone and that of ketorolac tromethamine alone, and the effect of reducing the side effects is better than that of naphazoline hydrochloride alone and that of ketorolac tromethamine alone.

The results above indicate that by combining the use of naphazoline hydrochloride and ketorolac tromethamine, it is possible to enhance the efficacy of pilocarpine nitrate while significantly reducing the side effects associated with pilocarpine nitrate.

### Experiment 2

According to Table 2-1-1, prepare the solution in the same way as Experiment 1 and conduct the efficacy test.

**Table 2-1-1: Examples of Different Pilocarpine Concentrations**

| Group | Pilocarpin e nitrate (weight% ) | Naphazol ine hydrochl oride (weight %) | Ketorolac trometha mine (weight% ) | Boric acid (weight% ) | Sodium citrate (weight% ) | Disodium edetate (weight% ) | Polyoxyet hylene 40 hydrogen ated castor oil (weight% ) |
|---|---|---|---|---|---|---|---|
| Example 1 | 1 | 0.05 | 0.5 | 1 | 0.015 | 0.015 | 0.5 |
| Example 2 | 0.5 | 0.05 | 0.5 | 1 | 0.015 | 0.015 | 0.5 |
| Example 3 | 0.1 | 0.05 | 0.5 | 1 | 0.015 | 0.015 | 0.5 |
| Example 4 | 1.5 | 0.05 | 0.5 | 1 | 0.015 | 0.015 | 0.5 |
| Example 5 | 3 | 0.05 | 0.5 | 1 | 0.015 | 0.015 | 0.5 |

### Experiment 2-1: Efficacy Test

The efficacy of the 5 formulations was evaluated using rabbit as the animal model, with the pupil diameter as the index. 30 rabbits were randomly divided into 5 groups, and one drop of test drug solution was applied to the right eyes of the rabbits in each group according to the Examples 1-5. The transverse pupil diameter was measured at different time points after administration using the anterior segment optical coherence tomography (anterior segment OCT).

The results are as follows:

**Table 2-1-2: Results of the pupil diameter after administration (mm)**

| Group | Before administr ation | 15min | 120min | 240min | 300min | 330min | 360min |
|---|---|---|---|---|---|---|---|
| Example 1 | 8.46 | 5.17 | 6.19 | 6.45 | 6.72 | 6.94 | 7.08 |
| Example 2 | 8.35 | 5.18 | 6.30 | 6.74 | 6.79 | 7.27 | 7.45 |
| Example 3 | 8.44 | 6.72 | 6.94 | 7.88 | 8.19 | 8.38 | 8.21 |
| Example 4 | 8.68 | 5.37 | 6.43 | 6.46 | 6.39 | 6.92 | 7.04 |
| Example 5 | 8.57 | 2.14 | 2.31 | 2.39 | 2.35 | 2.41 | 2.57 |

Through further calculation, the reduction value of pupil diameter after administration compared to before administration as follows.

**Table 2-1-3: the reduction in the pupil diameter after administration (mm)**

| Group | 15min | 120min | 240min | 300min | 330min | 360min |
|---|---|---|---|---|---|---|
| Example 1 | 3.29 | 2.27 | 2.01 | 1.74 | 1.52 | 1.38 |
| Example 2 | 3.17 | 2.05 | 1.61 | 1.56 | 1.08 | 0.9 |
| Example 3 | 1.72 | 1.5 | 0.56 | 0.25 | 0.06 | 0.23 |
| Example 4 | 3.31 | 2.25 | 2.22 | 2.29 | 1.76 | 1.64 |
| Example 5 | 6.43 | 6.26 | 6.18 | 6.22 | 6.16 | 6.00 |

According to the above test results, it can be known that:
In Example 1, 1 weight% of pilocarpine nitrate, 0.05 weight% of naphazoline hydrochloride, and 0.5 weight% of ketorolac tromethamine were used. The pupil diameter significantly decreased at 15 minutes after administration to the rabbit eyes, then slowly recovered, and remained effective at 6 hours after administration;
In Example 2, the same concentrations of naphazoline hydrochloride and ketorolac tromethamine were used as in Example 1, but the concentration of pilocarpine nitrate was reduced to 0.5%. The change in pupil diameter of the rabbit's eye after administration was essentially the same as in Example 1;
In Example 3, the same concentrations of naphazoline hydrochloride and ketorolac tromethamine were used as in Example 1, but the concentration of pilocarpine nitrate was reduced to 0.1%. The degree of reduction of the rabbit's eye pupil diameter after administration decreased compared to Example 1, and the pupil diameter of the rabbit's eye completely recovered after 4 hours;
In Example 4, the same concentrations of naphazoline hydrochloride and ketorolac tromethamine were used as in Example 1, but the concentration of pilocarpine nitrate increased to 1.5%. The change in pupil diameter of the rabbit's eye after administration was essentially the same as in Example 1;
In Example 5, the same concentrations of naphazoline hydrochloride and ketorolac tromethamine were used as in Example 1, but the concentration of pilocarpine nitrate increased to 3%. The pupil diameter of the rabbit's eye remained extremely reduced for 6 hours after administration.

The results of the above-mentioned tests indicate that the use of 0.5-1.5% pilocarpine nitrate can achieve the desired clinical effects and exhibits synergistic effects with naphazoline hydrochloride and ketorolac tromethamine in terms of efficacy. However, the use of 0.1% pilocarpine nitrate cannot meet the clinical requirements, and the use of 3% pilocarpine nitrate is also not suitable for clinical use. Therefore, in the present invention, it is preferred to use pilocarpine nitrate in the concentration range of 0.5-1.5%.

### Experiment 2-2:

According to Table 2-2-1, prepare the solution in the same way as Experiment 1-1 and conduct the irritation test.

**Table 2-2-1: Examples of Different Concentrations of Naphazoline and Ketorolac tromethamine**

| Group | Pilocar pine nitrate (weight %) | Naphazoli ne hydrochlo ride (weight%) | Ketorolac trometha mine (weight%) | Boric acid (weight %) | Sodium citrate (weight %) | Disodiu m edetate (weight %) | Polyoxyethy lene 40 hydrogenate d castor oil (weight%) |
|---|---|---|---|---|---|---|---|
| Example 1 | 1 | 0.05 | 0.5 | 1 | 0.015 | 0.015 | 0.5 |
| Example 6 | 1 | 0.005 | 0.5 | 1 | 0.015 | 0.015 | 0.5 |
| Example 7 | 1 | 0.0005 | 0.5 | 1 | 0.015 | 0.015 | 0.5 |
| Example 8 | 1 | 0.5 | 0.5 | 1 | 0.015 | 0.015 | 0.5 |
| Example 9 | 1 | 5 | 0.5 | 1 | 0.015 | 0.015 | 0.5 |
| Example 10 | 1 | 0.05 | 0.1 | 1 | 0.015 | 0.015 | 0.5 |
| Example 11 | 1 | 0.05 | 0.01 | 1 | 0.015 | 0.015 | 0.5 |
| Example 12 | 1 | 0.05 | 1 | 1 | 0.015 | 0.015 | 0.5 |
| Example 13 | 1 | 0.05 | 10 | 1 | 0.015 | 0.015 | 0.5 |

The irritation test was conducted on rabbits, using the degree of conjunctival congestion as an index to evaluate the aforementioned 9 formulations. 45 rabbits were randomly divided into 9 groups, and 1 drop of test drug solution of Examples 1, 6-13 was applied to the right eye of each rabbit in the respective groups. The degree of conjunctival congestion in the treated eyes was observed at different time points after administration, and the criteria for congestion assessment are shown in Table 1-2-2.

The results are as follows:

**Table 2-2-2: Example 1 Group**

| N | Before administr ation | 1min | 2min | 5min | 10min | 30min | 60min |
|---|---|---|---|---|---|---|---|
| 1 | 0 | 0 | 0 | 0 | 0 | 0 | 0 |
| 2 | 0 | 1 | 1 | 0 | 0 | 0 | 0 |
| 3 | 0 | 1 | 0 | 0 | 0 | 0 | 0 |
| 4 | 0 | 0 | 0 | 0 | 0 | 0 | 0 |
| 5 | 0 | 0 | 0 | 0 | 0 | 0 | 0 |
| average | 0 | 0.4 | 0.2 | 0 | 0 | 0 | 0 |

**Table 2-2-3: Example 6 Group**

| N | Before administr ation | 1min | 2min | 5min | 10min | 30min | 60min |
|---|---|---|---|---|---|---|---|
| 1 | 0 | 1 | 1 | 0 | 0 | 0 | 0 |
| 2 | 0 | 1 | 0 | 0 | 0 | 0 | 0 |
| 3 | 0 | 0 | 0 | 0 | 0 | 0 | 0 |
| 4 | 0 | 1 | 1 | 0 | 0 | 0 | 0 |
| 5 | 0 | 0 | 0 | 0 | 0 | 0 | 0 |
| average | 0 | 0.6 | 0.4 | 0 | 0 | 0 | 0 |

**Table 2-2-4: Example 7 Group**

| N | Before administr ation | 1min | 2min | 5min | 10min | 30min | 60min |
|---|---|---|---|---|---|---|---|
| 1 | 0 | 2 | 2 | 1 | 1 | 1 | 0 |
| 2 | 0 | 0 | 0 | 0 | 0 | 0 | 0 |
| 3 | 0 | 1 | 1 | 1 | 0 | 0 | 0 |
| 4 | 0 | 2 | 2 | 1 | 1 | 1 | 0 |
| 5 | 0 | 1 | 1 | 1 | 1 | 0 | 0 |
| average | 0 | 1 | 1.2 | 0.8 | 0.6 | 0.4 | 0 |

**Table 2-2-5: Example 8 Group**

| N | Before administr ation | 1min | 2min | 5min | 10min | 30min | 60min |
|---|---|---|---|---|---|---|---|
| 1 | 0 | 0 | 0 | 0 | 0 | 0 | 0 |
| 2 | 0 | 1 | 0 | 0 | 0 | 0 | 0 |
| 3 | 0 | 0 | 0 | 0 | 0 | 0 | 0 |
| 4 | 0 | 0 | 0 | 0 | 0 | 0 | 0 |
| 5 | 0 | 2 | 1 | 0 | 0 | 0 | 0 |
| average | 0 | 0.6 | 0.2 | 0 | 0 | 0 | 0 |

**Table 2-2-6: Example 9 Group**

| N | Before administr ation | 1min | 2min | 5min | 10min | 30min | 60min |
|---|---|---|---|---|---|---|---|
| 1 | 0 | 1 | 1 | 0 | 0 | 0 | 0 |
| 2 | 0 | 0 | 0 | 0 | 0 | 0 | 0 |
| 3 | 0 | 1 | 1 | 0 | 0 | 0 | 0 |
| 4 | 0 | 2 | 1 | 0 | 0 | 0 | 0 |
| 5 | 0 | 0 | 0 | 0 | 0 | 0 | 0 |
| average | 0 | 0.8 | 0.6 | 0 | 0 | 0 | 0 |

**Table 2-2-7: Example 10 Group**

| N | Before administr ation | 1min | 2min | 5min | 10min | 30min | 60min |
|---|---|---|---|---|---|---|---|
| 1 | 0 | 0 | 0 | 0 | 0 | 0 | 0 |
| 2 | 0 | 0 | 0 | 0 | 0 | 0 | 0 |
| 3 | 0 | 1 | 1 | 0 | 0 | 0 | 0 |
| 4 | 0 | 1 | 1 | 0 | 0 | 0 | 0 |
| 5 | 0 | 0 | 0 | 0 | 0 | 0 | 0 |
| average | 0 | 0.4 | 0.4 | 0 | 0 | 0 | 0 |

**Table 2-2-8: Example 11 Group**

| N | Before administr ation | 1min | 2min | 5min | 10min | 30min | 60min |
|---|---|---|---|---|---|---|---|
| 1 | 0 | 0 | 0 | 0 | 0 | 0 | 0 |
| 2 | 0 | 1 | 1 | 0 | 0 | 0 | 0 |
| 3 | 0 | 1 | 1 | 1 | 1 | 0 | 0 |
| 4 | 0 | 1 | 1 | 1 | 0 | 0 | 0 |
| 5 | 0 | 1 | 1 | 1 | 1 | 0 | 0 |
| average | 0 | 0.8 | 0.8 | 0.6 | 0.4 | 0 | 0 |

**Table 2-2-9: Example 12 Group**

| N | Before administr ation | 1min | 2min | 5min | 10min | 30min | 60min |
|---|---|---|---|---|---|---|---|
| 1 | 0 | 1 | 1 | 0 | 0 | 0 | 0 |
| 2 | 0 | 0 | 0 | 0 | 0 | 0 | 0 |
| 3 | 0 | 1 | 1 | 0 | 0 | 0 | 0 |
| 4 | 0 | 1 | 1 | 0 | 0 | 0 | 0 |
| 5 | 0 | 0 | 0 | 0 | 0 | 0 | 0 |
| average | 0 | 0.6 | 0.6 | 0 | 0 | 0 | 0 |

**Table 2-2-10: Example 13 Group**

| N | Before administr ation | 1min | 2min | 5min | 10min | 30min | 60min |
|---|---|---|---|---|---|---|---|
| 1 | 0 | 2 | 1 | 1 | 1 | 1 | 0 |
| 2 | 0 | 1 | 1 | 0 | 0 | 0 | 0 |
| 3 | 0 | 1 | 1 | 1 | 0 | 0 | 0 |
| 4 | 0 | 2 | 1 | 1 | 1 | 0 | 0 |
| 5 | 0 | 2 | 1 | 1 | 1 | 1 | 0 |
| average | 0 | 1.6 | 1 | 0.8 | 0.6 | 0.4 | 0 |

Based on the results of the above tests, it can be concluded that:
In Example 1, 1 weight% of pilocarpine nitrate, 0.05 weight% of naphazoline hydrochloride, and 0.5 weight% of ketorolac tromethamine were used. Among the 5 animals, 2 showed mild conjunctival congestion at 1 min after administration, which resolved after 2 min. The results were consistent with those in Experiment 1-2;
In Example 6, the same concentrations of pilocarpine nitrate and ketorolac tromethamine were used as in Example 1, but the concentration of naphazoline hydrochloride was reduced to 0.005 weight%. Among the 5 animals, 3 showed mild conjunctival congestion after administration, which resolved after 2 minutes. The test results were generally similar to those of Example 1;
In Example 7, the same concentrations of pilocarpine nitrate and ketorolac tromethamine were used as in Example 1, but the concentration of naphazoline hydrochloride was reduced to 0.0005 weight%. Among the 5 animals, 4 showed mild to moderate conjunctival congestion after administration, which lasted for 30 min. The test results were significantly inferior than those in Example 1;
In Example 8, the same concentrations of pilocarpine nitrate and ketorolac tromethamine were used as in Example 1, but the concentration of naphazoline hydrochloride was increased to 0.5 weight%. Among the 5 animals, 2 showed mild to moderate conjunctival congestion, which resolved after 2 minutes. The test results were generally similar to those of Example 1;
In Example 9, the same concentrations of pilocarpine nitrate and ketorolac tromethamine were used as in Example 1, but the concentration of naphazoline hydrochloride was increased to 5 weight%. Among the 5 animals, 3 showed mild to moderate conjunctival congestion, which resolved after 2 min. The test results were inferior to those of Example 1;
In Example 10, the same concentrations of pilocarpine nitrate and naphazoline hydrochloride were used as in Example 1, but the concentration of ketorolac tromethamine was reduced to 0.1 weight%. Among the 5 animals, 2 showed mild conjunctival congestion after administration, which resolved after 2 minutes. The test results were similar to those of Example 1;
In Example 11, the same concentrations of pilocarpine nitrate and naphazoline hydrochloride were used as in Example 1, but the concentration of ketorolac tromethamine was reduced to 0.01 weight%. Among the 5 animals, 4 showed mild conjunctival congestion after administration, which lasted for more than 10 minutes. The test results were inferior to those of Example 1;
In Example 12, the same concentrations of pilocarpine nitrate and naphazoline hydrochloride were used as in Example 1, but the concentration of ketorolac tromethamine was increased to 1 weight%. Among the 5 animals, 3 showed mild conjunctival congestion after administration, which resolved after 2 minutes. The test results were generally the same as those in Example 6;
In Example 13, the same concentrations of pilocarpine nitrate and naphazoline hydrochloride were used as in Example 1, but the ketorolac tromethamine concentration was increased to 10 weight%. 5 animals all showed mild to moderate conjunctival congestion after administration, which lasted for more than 30 minutes. The test results were inferior than those in Example 1.

The above-mentioned test results show that when naphazoline hydrochloride in the concentration range of 0.005-0.5% is used in combination with pilocarpine nitrate and ketorolac tromethamine, good irritation reduction effect can be achieved. In addition, when 0.0005 weight% of naphazoline hydrochloride is used, the irritation reduction effect is insufficient. Moreover, when 5 weight% of naphazoline hydrochloride is used, the irritation reduction effect is weakened. Therefore, in the present invention, it is preferred to use naphazoline hydrochloride in the concentration range of 0.005-0.5%.

In addition, the above test results also show that when ketorolac tromethamine in the concentration range of 0.1-1% is used in combination with pilocarpine nitrate and naphazoline hydrochloride, good irritation reduction effect can be achieved. In addition, when using ketorolac tromethamine in the concentration of 0.01 weight%, the irritation reduction effect is insufficient. In addition, when using ketorolac tromethamine in the concentration of 10 weight%, the irritation reduction effect is insufficient. Therefore, in the present invention, it is preferred to use ketorolac tromethamine in the concentration range of 0.1-1%.

### Experiment 3

According to Table 3-1, use other α receptor agonists and nonsteroidal anti-inflammatory drugs (NSAIDs) to prepare the solution in the same way as Experiment 1-1, and conduct efficacy and irritation tests.

**Table 3-1: Examples of the Use of Other α Receptor Agonists and Nonsteroidal Anti-inflammatory Drugs**

| Group | Acetylch olin e (wei ght %) | Carbachol (wei ght% ) | Pilocarpin e nitrat e (wei ght% ) | α receptor agonists (weight%) | | Nonsteroidal anti-inflammatory drugs (weight%) | | | Boric acid (weig ht%) | Sodium citrate (weigh t%) | Disodium edetate (weight %) | Polyoxy ethylene 40 hydroge nated castor oil (weight %) |
|---|---|---|---|---|---|---|---|---|---|---|---|---|
| | | | | Naph azoli ne hydr ochlo ride | Oxym etazol ine hydro chlori de | Keto rolac trom etha mine | Dicl ofen ac | Pran opro fen | | | | |
| Exam ple 1 | | | 1 | 0.05 | 0 | 0.5 | 0 | 0 | 1 | 0.015 | 0.015 | 0.5 |
| Comp arativ e Exam ple 4 | | | 1 | 0.05 | 0 | 0 | 0.5 | 0 | 1 | 0.015 | 0.015 | 0.5 |
| Comp arativ e Exam ple 5 | | | 1 | 0.05 | 0 | 0 | 0 | 0.5 | 1 | 0.015 | 0.015 | 0.5 |
| Comp arativ e Exam ple 6 | | | 1 | 0 | 0.05 | 0 | 0 | 0.5 | 1 | 0.015 | 0.015 | 0.5 |
| Comp arativ e Exam ple 7 | | | 1 | 0 | 0.05 | 0.5 | 0 | 0 | 1 | 0.015 | 0.015 | 0.5 |
| Comp arativ e Exam ple 8 | | | 1 | 0 | 0.05 | 0 | 0.5 | 0 | 1 | 0.015 | 0.015 | 0.5 |
| Comp arativ e Exam ple 9 | 1 | | | 0.05 | | 0.5 | | | 1 | 0.015 | 0.015 | 0.5 |
| Comp arativ e Exam ple 10 | | 1 | | 0.05 | | 0.5 | | | 1 | 0.015 | 0.015 | 0.5 |

It should be noted that in the above examples, the Comparative Examples 4 and 8 showed drug precipitation during formulation, indicating that the druggability of diclofenac and pilocarpine nitrate was poor, and thus the Comparative Examples 4 and 8 were not prepared into final formulations.

The 6 formulations obtained in Example 1, Comparative Examples 5-7, and Comparative Examples 9-10 were evaluated for efficacy and irritation using rabbits. 30 rabbits were randomly divided into 6 groups, and one drop of test drug solution was applied to the right eyes of the rabbits in each group according to the Examples 1, Comparative Examples 5-7, and Comparative Examples 9-10.

### Experiment 3-1: Efficacy Test

Using the pupil diameter of the rabbit's eye as an index, the transverse pupil diameter was measured at different time points after administration using the anterior segment optical coherence tomography (anterior segment OCT).

The results are as follows:

**Table 3-2: Results of pupil diameter after administration (mm)**

| Group | Before administr ation | 15min | 120min | 240min | 300min | 330min | 360min |
|---|---|---|---|---|---|---|---|
| Example 1 | 8.46 | 5.17 | 6.19 | 6.45 | 6.72 | 6.94 | 7.08 |
| Comparati ve Example 5 | 8.52 | 5.14 | 6.41 | 6.89 | 7.26 | 7.98 | 8.26 |
| Comparati ve Example 6 | 8.44 | 5.36 | 6.57 | 7.19 | 7.45 | 8.15 | 8.37 |
| Comparati ve Example 7 | 8.72 | 5.79 | 6.84 | 7.07 | 7.51 | 8.32 | 8.66 |
| Comparati ve Example 9 | 8.61 | 6.54 | 6.99 | 7.27 | 7.73 | 8.18 | 8.46 |
| Comparati ve Example 10 | 8.55 | 6.87 | 7.25 | 7.48 | 7.83 | 8.21 | 8.39 |

Based on the results of the above tests, it can be concluded that:
In Example 1, 1 weight% of pilocarpine nitrate, 0.05 weight% of naphazoline hydrochloride, and 0.5 weight% of ketorolac tromethamine were used. The pupil diameter significantly decreased at 15 minutes after administration to the rabbit eyes, then slowly recovered, and remained effective at 6 hours after administration. The results of this experiment were consistent with Experiment 1-1;
Comparative Example 5 used pilocarpine nitrate and naphazoline hydrochloride at the same concentrations as Example 1, and replaced ketorolac tromethamine with 0.5 weight% of pranoprofen. The test results of changes in the pupil diameter of the rabbit's eye after administration were significantly inferior than those of Example 1;
Comparative Example 6 used pilocarpine nitrate with the same concentration as Example 1, and replaced naphazoline hydrochloride with 0.05 weight% of oxymetazoline hydrochloride, and replaced ketorolac tromethamine with 0.5 weight% of pranoprofen. The test results of the change in pupil diameter of the rabbit's eye after administration were significantly inferior than those of Example 1.

Comparative Example 7 used pilocarpine nitrate and naphazoline hydrochloride at the same concentrations as Example 1, and replaced ketorolac tromethamine with 0.5 weight% of pranoprofen. The test results of changes in the pupil diameter of the rabbit's eye after administration were significantly inferior than those of Example 1.

Comparative Examples 9 and 10, using other M3 receptor agonists, showed that none of them could achieve the effect achieved by pilocarpine nitrate.

The efficacy test results show that only when ketorolac tromethamine is used in combination with naphazoline hydrochloride can a synergistic effect be achieved in terms of efficacy. When other α receptor agonists or nonsteroidal anti-inflammatory drugs are used, this synergistic effect cannot be achieved, or the ingredients cannot be made into preparation and become medicine.

### Experiment 3-2: Irritation Test

Using the degree of conjunctival congestion as an index, observe the degree of conjunctival congestion in the treated eye at different time points after administration. The evaluation criteria for congestion severity are shown in Table 1-2-1.

The results are as follows:

**Table 3-3: Example 1 Group**

| N | Before administr ation | 1min | 2min | 5min | 10min | 30min | 60min |
|---|---|---|---|---|---|---|---|
| 1 | 0 | 0 | 0 | 0 | 0 | 0 | 0 |
| 2 | 0 | 0 | 0 | 0 | 0 | 0 | 0 |
| 3 | 0 | 0 | 0 | 0 | 0 | 0 | 0 |
| 4 | 0 | 1 | 1 | 0 | 0 | 0 | 0 |
| 5 | 0 | 0 | 0 | 0 | 0 | 0 | 0 |
| average | 0 | 0.2 | 0.2 | 0 | 0 | 0 | 0 |

**Table 3-4: Comparative Example 5 Group**

| N | Before administr ation | 1min | 2min | 5min | 10min | 30min | 60min |
|---|---|---|---|---|---|---|---|
| 1 | 0 | 1 | 1 | 1 | 0 | 0 | 0 |
| 2 | 0 | 1 | 0 | 0 | 0 | 0 | 0 |
| 3 | 0 | 0 | 0 | 0 | 0 | 0 | 0 |
| 4 | 0 | 1 | 1 | 1 | 1 | 1 | 0 |
| 5 | 0 | 1 | 1 | 1 | 1 | 0 | 0 |
| average | 0 | 0.8 | 0.6 | 0.6 | 0.4 | 0 | 0 |

**Table 3-5: Comparative Example 6 Group**

| N | Before administr ation | 1min | 2min | 5min | 10min | 30min | 60min |
|---|---|---|---|---|---|---|---|
| 1 | 0 | 1 | 1 | 1 | 1 | 0 | 0 |
| 2 | 0 | 1 | 1 | 1 | 0 | 0 | 0 |
| 3 | 0 | 0 | 0 | 0 | 0 | 0 | 0 |
| 4 | 0 | 2 | 1 | 1 | 1 | 1 | 0 |
| 5 | 0 | 0 | 0 | 0 | 0 | 0 | 0 |
| average | 0 | 0.8 | 0.6 | 0.6 | 0.4 | 0.2 | 0 |

**Table 3-6: Comparative Example 7 Group**

| N | Before administr ation | 1min | 2min | 5min | 10min | 30min | 60min |
|---|---|---|---|---|---|---|---|
| 1 | 0 | 1 | 1 | 1 | 1 | 0 | 0 |
| 2 | 0 | 1 | 1 | 1 | 0 | 0 | 0 |
| 3 | 0 | 0 | 0 | 0 | 0 | 0 | 0 |
| 4 | 0 | 1 | 1 | 1 | 0 | 1 | 0 |
| 5 | 0 | 0 | 0 | 0 | 0 | 0 | 0 |
| average | 0 | 0.6 | 0.6 | 0.6 | 0.2 | 0.2 | 0 |

**Table 3-7: Comparative Example 9 Group**

| N | Before administr ation | 1min | 2min | 5min | 10min | 30min | 60min |
|---|---|---|---|---|---|---|---|
| 1 | 0 | 1 | 0 | 0 | 0 | 0 | 0 |
| 2 | 0 | 1 | 1 | 1 | 0 | 0 | 0 |
| 3 | 0 | 1 | 1 | 1 | 0 | 0 | 0 |
| 4 | 0 | 2 | 1 | 1 | 1 | 1 | 0 |
| 5 | 0 | 0 | 0 | 0 | 0 | 0 | 0 |
| average | 0 | 1.0 | 0.6 | 0.6 | 0.2 | 0.2 | 0 |

**Table 3-8: Comparative Example 10 Group**

| N | Before administr ation | 1min | 2min | 5min | 10min | 30min | 60min |
|---|---|---|---|---|---|---|---|
| 1 | 0 | 0 | 0 | 0 | 0 | 0 | 0 |
| 2 | 0 | 1 | 1 | 0 | 0 | 0 | 0 |
| 3 | 0 | 1 | 1 | 1 | 0 | 0 | 0 |
| 4 | 0 | 1 | 1 | 0 | 0 | 0 | 0 |
| 5 | 0 | 2 | 1 | 1 | 1 | 1 | 0 |
| average | 0 | 1.0 | 0.8 | 0.4 | 0.2 | 0.2 | 0 |

Based on the results of the above tests, it can be concluded that:
In Example 1, pilocarpine nitrate, naphazoline hydrochloride, and ketorolac tromethamine were used in combination. Among the 5 animals, 1 showed mild conjunctival congestion 1 min after administration, which resolved after 2 min. The test results were consistent with those of Experiment 1-2;
In Comparative Example 5, pilocarpine nitrate and naphazoline hydrochloride were used, and pranoprofen was used instead of ketorolac tromethamine. Among the 5 animals, 4 showed mild conjunctival congestion after administration and lasting for 30 min. The test results were significantly inferior than those in Example 1.

In Comparative Example 6, pilocarpine nitrate was used, and instead of naphazoline hydrochloride, oxymetazoline hydrochloride was used, and instead of ketorolac tromethamine, pranoprofen was used. Among the 5 animals, 3 showed mild to moderate congestion after administration and lasting for 30 min. The test results were significantly inferior than those of Example 1.

Comparative Example 7 used pilocarpine nitrate and naphazoline hydrochloride, and 0.5 weight% of pranoprofen was used instead of ketorolac tromethamine. Among the 5 animals, 3 showed mild congestion after administration and lasting for 30 min. The test results were significantly inferior than those of Example 1.

In Comparative Examples 9 and 10, other M3 receptor agonists were used instead of pilocarpine nitrate. The results showed that in both groups of experiments, 4 of 5 animals showed mild to moderate congestion after administration and lasting for 30 min. The test results were significantly inferior than those of Example 1.

The results of the above irritation test indicate that only when naphazoline hydrochloride is used in combination with ketorolac tromethamine can a synergistic effect be achieved, minimizing the irritation caused by pilocarpine nitrate and achieving the optimal reduction in the side effects of pilocarpine nitrate. In addition, when other α receptor agonists such as oxymetazoline hydrochloride or other nonsteroidal anti-inflammatory drugs (NSAIDS) such as diclofenac or pranoprofen are used, the reduction in the side effects of pilocarpine nitrate is significantly insufficient, meaning that a synergistic effect cannot be achieved, or the ingredients cannot be made into preparation and become medicine.

### Experiment 4

**Table 4-1: Examples of Using Other Muscarinic Acetylcholine Receptor M3 Agonists**

| Gro up | Pilocarpine (%) | | | Napha zoline hydro chlori de(%) | Ketorol ac trometh amine( %) | Boric acid (%) | Sodiu m citrate (%) | disodi um edetat e (%) | Polyoxye thylene 40 hydrogen ated castor oil (%) | Sodiu m hydro xide and/or citric acid are used to adjust pH to |
|---|---|---|---|---|---|---|---|---|---|---|
| | Piloca rpine | Pilocarpi ne hydrochl oride | Pilocarpi ne nitrate | | | | | | | |
| Exa mple 14 | 1.1 | 0 | 0 | 0.02 | 0.6 | 1 | 0.015 | 0.015 | 0.5 | 5.0 |
| Exa mple 15 | 0 | 1.25 | 0 | 0.1 | 0.5 | 1 | 0.015 | 0.015 | 0.5 | 5.0 |
| Exa mple 16 | 0 | 0 | 1.38 | 0.05 | 0.4 | 1 | 0.015 | 0.015 | 0.5 | 5.0 |
| Exa mple 17 | 1.1 | 0 | 0 | 0.05 | 0.4 | 1 | 0.015 | 0.015 | 0.5 | 5.0 |
| Exa mple 18 | 0 | 1.25 | 0 | 0.05 | 0.4 | 1 | 0.015 | 0.015 | 0.5 | 5.0 |

According to Table 4-1, use other muscarinic acetylcholine receptor M3 agonists, prepare the solution in the same way as in Experiment 1-1, and conduct efficacy and irritation tests.

It should be noted that, from the perspective of using the same molar amount of pilocarpine, the use of 1.25% pilocarpine hydrochloride and 1.38% pilocarpine nitrate is equivalent to using 1.1% pilocarpine.

During the efficacy and irritation tests, 20 rabbits were randomly divided into 4 groups, and one drop of test drug solution was applied to the right eye of each rabbit in the respective group according to Examples 14-16.

### Experiment 4-1: Efficacy Test

Efficacy validation was conducted using pupil diameter as an index. The lateral pupil diameter was measured at different time points after administration using the anterior segment optical coherence tomography (anterior segment OCT).

The following is the result of the reduction in lateral pupil diameter after administration compared to before administration:

**Table 4-2: Results of reduction in pupil diameter after administration (mm)**

| Group | 15min | 120min | 240min | 300min | 330min | 360min |
|---|---|---|---|---|---|---|
| Example 14 | 2.88 | 1.65 | 1.40 | 1.19 | 1.06 | 0.87 |
| Example 15 | 3.01 | 1.79 | 1.54 | 1.33 | 1.28 | 1.06 |
| Example 16 | 3.62 | 2.44 | 2.18 | 2.02 | 1.90 | 1.69 |
| Example 17 | 3.43 | 2.27 | 2.01 | 1.81 | 1.69 | 1.55 |
| Example 18 | 3.56 | 2.38 | 2.12 | 1.97 | 1.82 | 1.63 |

### Test 4-2: Irritation Test

Conduct irritation verification using the degree of conjunctival congestion as an index. Observe the degree of conjunctival congestion in the treated eye at different time points after administration, and the evaluation criteria for congestion severity are shown in Table 1-2-2.

The results are as follows:

**Table 4-3: Example 14 Group**

| N | Before administr ation | 1min | 2min | 5min | 10min | 30min | 60min |
|---|---|---|---|---|---|---|---|
| 1 | 0 | 1 | 1 | 0 | 0 | 0 | 0 |
| 2 | 0 | 0 | 0 | 0 | 0 | 0 | 0 |
| 3 | 0 | 1 | 1 | 0 | 0 | 0 | 0 |
| 4 | 0 | 0 | 0 | 0 | 0 | 0 | 0 |
| 5 | 0 | 0 | 0 | 0 | 0 | 0 | 0 |
| average | 0 | 0.4 | 0.4 | 0 | 0 | 0 | 0 |

**Table 4-4: Example 15 Group**

| N | Before administr ation | 1min | 2min | 5min | 10min | 30min | 60min |
|---|---|---|---|---|---|---|---|
| 1 | 0 | 0 | 0 | 0 | 0 | 0 | 0 |
| 2 | 0 | 0 | 0 | 0 | 0 | 0 | 0 |
| 3 | 0 | 1 | 1 | 0 | 0 | 0 | 0 |
| 4 | 0 | 1 | 0 | 0 | 0 | 0 | 0 |
| 5 | 0 | 0 | 0 | 0 | 0 | 0 | 0 |
| average | 0 | 0.4 | 0.2 | 0 | 0 | 0 | 0 |

**Table 4-5: Example 16 Group**

| N | Before administr ation | 1min | 2min | 5min | 10min | 30min | 60min |
|---|---|---|---|---|---|---|---|
| 1 | 0 | 0 | 0 | 0 | 0 | 0 | 0 |
| 2 | 0 | 0 | 0 | 0 | 0 | 0 | 0 |
| 3 | 0 | 0 | 0 | 0 | 0 | 0 | 0 |
| 4 | 0 | 0 | 0 | 0 | 0 | | 0 |
| 5 | 0 | 1 | 1 | 0 | 0 | 0 | 0 |
| average | 0 | 0.2 | 0.2 | 0 | 0 | 0 | 0 |

**Table 4-6: Example 17 Group**

| N | Before administr ation | 1min | 2min | 5min | 10min | 30min | 60min |
|---|---|---|---|---|---|---|---|
| 1 | 0 | 0 | 0 | 0 | 0 | 0 | 0 |
| 2 | 0 | 0 | 0 | 0 | 0 | 0 | 0 |
| 3 | 0 | 0 | 0 | 0 | 0 | 0 | 0 |
| 4 | 0 | 0 | 0 | 0 | 0 | | 0 |
| 5 | 0 | 1 | 0 | 0 | 0 | 0 | 0 |
| average | 0 | 0.2 | 0.2 | 0 | 0 | 0 | 0 |

**Table 4-7: Example 18 Group**

| N | Before administr ation | 1min | 2min | 5min | 10min | 30min | 60min |
|---|---|---|---|---|---|---|---|
| 1 | 0 | 0 | 0 | 0 | 0 | 0 | 0 |
| 2 | 0 | 0 | 0 | 0 | 0 | 0 | 0 |
| 3 | 0 | 0 | 0 | 0 | 0 | 0 | 0 |
| 4 | 0 | 1 | 1 | 0 | 0 | | 0 |
| 5 | 0 | 0 | 0 | 0 | 0 | 0 | 0 |
| average | 0 | 0.2 | 0.2 | 0 | 0 | 0 | 0 |

The results of the tests in Examples 14-16 above show that, in the case where the same number of moles of pilocarpine is used in the present invention, the technical effects of the present invention can be achieved when each component is used at an optional concentration within the specific concentration range of naphazoline hydrochloride (%) and ketorolac tromethamine (%).

The results of the tests in Examples 16-18 above indicate that, the technical effects of the present invention also can be achieved with other pharmaceutically acceptable salt of pilocarpine instead of pilocarpine nitrate. When using pilocarpine nitrate, pharmacodynamic effects superior to those of other salt can be achieved, and equivalent safety can be maintained.

## Claims

1. A composition comprising, relative to the total weight of the composition:
0.5-1.5 weight% of muscarinic acetylcholine receptor M3 agonist;
0.005-0.5 weight% of naphazoline, its pharmaceutically acceptable salt, or a combination thereof; and
0.1-1 weight% of ketorolac tromethamine.

2. The composition of claim 1, wherein the muscarinic acetylcholine receptor M3 agonist comprises at least one selected from the group consisting of acetylcholine, bethanechol chloride, carbachol, oxotremorine, pilocarpidine, pilocarpine, a pharmaceutically acceptable salt thereof, or a combination thereof;
preferably, the muscarinic acetylcholine receptor M3 agonist comprises at least one selected from pilocarpine, its pharmaceutically acceptable salt, or a combination thereof;
preferably, the muscarinic acetylcholine receptor M3 agonist is pilocarpine, pilocarpine hydrochloride, pilocarpine nitrate, or a combination thereof, preferably pilocarpine nitrate.

3. The composition of claim 1 or 2, wherein the pharmaceutically acceptable salt of naphazoline is naphazoline hydrochloride.

4. The composition of any one of claims 1-3, wherein the composition has a pH of 3.0-8.5 at room temperature, preferably the composition has a pH of 4.0-6.0 at room temperature, and preferably the composition has a pH of 4.5-5.5 at room temperature.

5. The composition of any one of claims 1-4, wherein the composition further comprises boric acid, preferably in an amount of 0.5-1.5 weight% relative to the total weight of the composition.

6. The composition of any one of claims 1-5, wherein the composition further comprises a surfactant;
preferably, the surfactant is 0.1-1 weight% relative to the total weight of the composition;
preferably, the surfactant comprises one or more selected from the group consisting of polyoxyethylene (20) hydrogenated castor oil, polyoxyethylene (40) hydrogenated castor oil, polyoxyethylene (60) hydrogenated castor oil, polysorbate 80, Span, poloxamer, propylene glycol, tyloxapol, and nonoxynol;
preferably, the surfactant is polyoxyethylene (40) hydrogenated castor oil.

7. The composition of any one of claims 1-6, wherein the composition further comprises:
0.005-0.05 weight% of sodium citrate, relative to the total weight of the composition, and/or
0.005-0.05 weight% of disodium edetate relative to the total weight of the composition.

8. The composition of any one of claims 1-7, wherein the composition further comprises:
0-0.1 weight% of citric acid and/or 0-0.1 weight% of sodium hydroxide relative to the total weight of the composition.

9. The composition of any one of claims 1-8, wherein the composition comprises:
0.5-1.5 weight% of pilocarpine nitrate;
0.005-0.5 weight% of naphazoline and/or naphazoline hydrochloride;
0.1-1 weight% of ketorolac tromethamine;
0.5-1.5 weight% of boric acid;
0.005-0.05 weight% of sodium citrate;
0.005-0.05 weight% of disodium edetate;
0.1-1 weight% of polyoxyethylene (40) hydrogenated castor oil;
0-0.1 weight% of citric acid;
0-0.1 weight% of sodium hydroxide; and
remaining injection water.

10. Use of the composition of any one of claims 1-9 in the preparation of a medicament for the treatment of ophthalmic disease, such as presbyopia.
